(19) Europäisches Patentamt
European Patent Office
Office européen des brevets

(11) Publication number: 0 018 231
B1

(12) EUROPEAN PATENT SPECIFICATION

(45) Date of publication of patent specification: 20.07.83

(51) Int. Cl.³: C 07 D 519/04, A 61 K 31/395

(21) Application number: 80301290.5

(22) Date of filing: 22.04.80

# (54) A process for the preparation of bis-indole-alkaloids and acid addition salts thereof and new bis-indole-alkaloids, pharmaceutical compositions containing them and their use as antitumour agents.

(30) Priority: 23.04.79 HU RI000708
30.11.79 HU RI000708

(43) Date of publication of application:
29.10.80 Bulletin 80/22

(45) Publication of the grant of the patent:
20.07.83 Bulletin 83/29

(84) Designated Contracting States:
AT BE CH DE FR GB IT LI LU NL SE

(56) References cited:
GB - A - 1 412 932
US - A - 3 899 493

(73) Proprietor: RICHTER GEDEON VEGYESZETI GYAR R.T.
Gyömröi ut 19-21
H-1475 Budapest X (HU)

(72) Inventor: Jovanovics, Karola
Detreko u. 3/a
1022 Budapest (HU)
Inventor: Gorog, Sandor
Vajda Peter u. 43/B
1089 Budapest (HU)
Inventor: Eckhardt, Sandor
Julia u. 1.
1026 Budapest (HU)
Inventor: Sugar, Janos
Matroz u. 6
1035 Budapest (HU)
Inventor: Somfai, Zsuzsa
Oktober 6. u. 16
1051 Budapest (HU)
Inventor: Farkas, Elek
Koer u. 44
1103 Budapest (HU)
Inventor: Hindy, Ivan
Kelemen Laszlo u. 9
1026 Budapest (HU)

EP 0 018 231 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

(74) Representative: **Woodman, Derek et al,**
**FRANK B. DEHN & CO. Imperial House 15-19**
**Kingsway**
**London WC2B 6UZ (GB)**

# A process for the preparation of bis-indole-alkaloids and acid addition salts thereof and new bis-indole-alkaloids, pharmaceutical compositions containing them and their use as antitumour agents

The present invention relates to a process for the preparation of bis-indole-alkaloids and acid addition salts thereof, as well as to new bis-indole-alkaloids. More particularly, the invention concerns the oxidation of the N-methyl group of vinblastine, leurosine or an acid addition salt thereof by chromic acid ($CrO_3$) or a salt thereof.

Vinblastine and leurosine may be described by the general formula II,

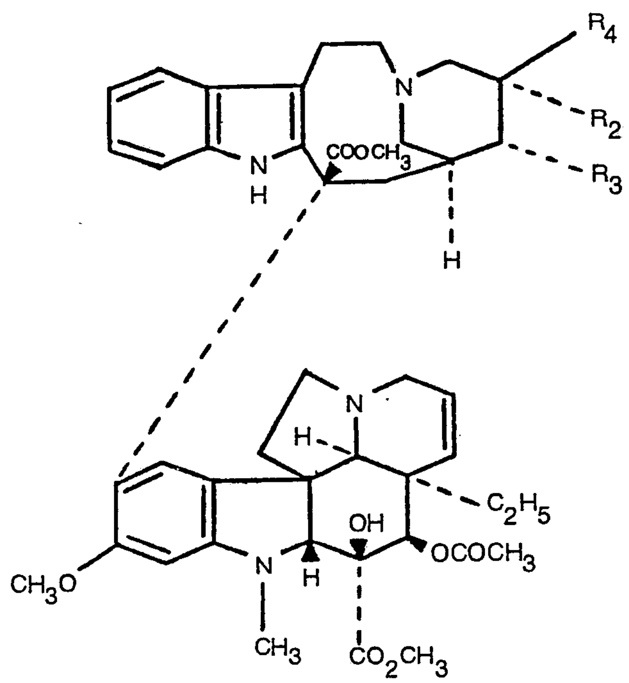

(II)

whereby in vinblastine $R_2$ represents an ethyl group, $R_3$ represents a hydrogen atom and $R_4$ represents a hydroxy group and in leurosine $R_2$ and $R_3$ together represent an epoxy group and $R_4$ represents an ethyl group.

Vinblastine and acid addition salts thereof are for example described in U.S. Patent Specification Ser. No. 3,097,137, while leurosine is the subject of U.S. Patent Specification Ser. No. 3,370,057. Vinblastine is used as an antitumour agent in clinical practice, the trade name of compositions containing vinblastine being Velbe. The antitumour activity of leurosine has also been shown in animals but it cannot successfully be used in human therapy due to its unreasonably high toxicity on the hematopoietic organs (Rohn, R. *et al.* Cancer Chemother. Reports *38,* 53 [1963]).

The preparation of vincristine (N-desmethyl-N-formyl-vinblastine) is for example described in U.S. Patent Specification No. 3,205,220, in Belgian Patent Specification No. 819,663, in Hungarian Patent Specifications Nos. 168,433 and 165,599 (corresponding to U.S. Patent Specification No. 3,899,493) and in German Patent Specification No. 2,614,863; and N-desmethyl-vinblastine can be prepared according to U.S. Patent Specification No. 3,354,163, Belgian Patent Specification No. 819,078 and Hungarian Patent Specification No. 165,599. N-desmethyl-N-formyl-leurosine and N-desmethyl-leurosine are disclosed in Hungarian Patent Specification No. 165,986 (corresponding to British Patent Specification No. 1,412,932). The N-desmethyl derivatives have no notable antitumour activity. The N-formyl derivatives are primarily used in the control of acute leukemia and various lymphomic diseases; while vincristine is used in the treatment of psoriasis (see U.S. Patent Specification No. 3,749,784).

Since the use of these known diindole-alkaloids is accompanied by toxic side effects — for instance the administration of vincristine induces paralytic symptoms due to neurotoxicity — a considerable effort has been made to prepare new derivatives having the same or similar activity without or with less undesired toxic effects. Diindole-alkaloids which cannot themselves be directly utilized for medical purposes are often converted into pharmacologically utilizable alkaloids.

For example according to the process disclosed in Hungarian Patent Specification No. 165,986 leurosine is converted into the effective, non-toxic N-desmethyl-N-formyl-leurosine; or according to Hungarian Patent Specification No. 165,599 vinblastine is transformed into the more effective N-formyl derivative, i.e. vincristine. According to these processes the starting compound is oxidized with chromium trioxide in a mixture of glacial acetic acid, acetone and acetic anhydride at a temperature

between −90°C and −30°C. Thereafter the reaction mixture is neutralised and from the mixture obtained the N-desmethyl and N-desmethyl-N-formyl derivatives may be isolated, or, if desired, the N-desmethyl derivative may be converted into the corresponding N-desmethyl-N-formyl derivative by a suitable formylating agent.

It is an object of the present invention to provide a new oxidation process and new vinblastine and leurosine derivatives.

We have now surprisingly found that a considerable advantage results if, in the process described in Hungarian Patent Specification Nos. 165,986 and 165,599, the acetone is replaced by a water-immiscible organic solvent in which both the alkaloid starting materials and the alkaloid products are readily soluble, (preferably a chlorinated hydrocarbon) and a small amount of ethanol. When the oxidation is effected in this way, new derivatives are obtained as well as the expected products, which derivatives have shown interesting pharmacological properties.

Thus according to one feature of the present invention there is provided a process for the preparation of alkaloids of the formula I

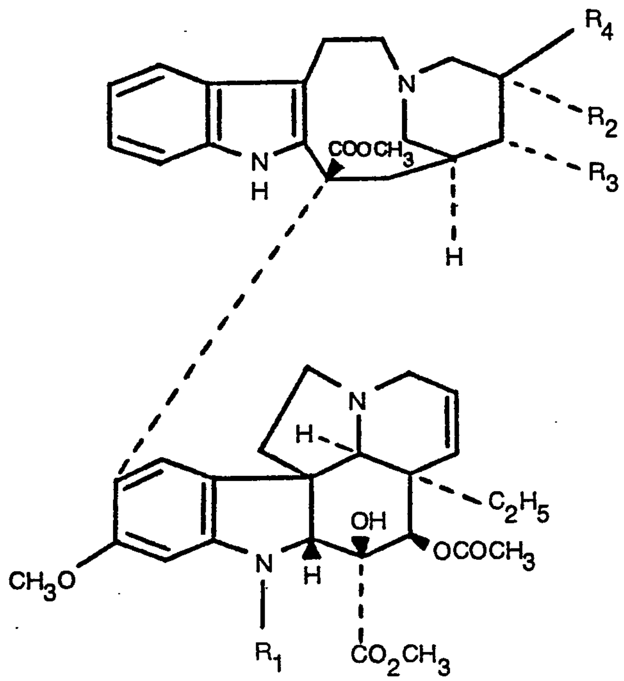

(I)

(wherein $R_1$ represents a hydrogen atom or a formyl group and *either* $R_2$ represents an ethyl group, $R_3$ represents a hydrogen atom and $R_4$ represents a hydroxy group *or* $R_2$ and $R_3$ together represent an epoxy group and $R_4$ represents an ethyl group) and acid addition salts thereof which comprises reacting an alkaloid of formula II as hereinbefore defined or an acid addition salt thereof with chromic acid or an alkali metal dichromate, at a temperature of from −30°C to −90°C in the presence of a water-immiscible organic solvent in which the alkaloid starting materials and alkaloid products are readily soluble, acetic anhydride and optionally glacial acetic acid, the reaction medium also containing ethanol in an amount of from 0.1 to 10% by volume based on the volume of the water-immiscible organic solvent; thereafter adjusting the pH of the reaction mixture to from 8 to 10; and isolating the products obtained and/or subjecting the said products to further treatment; whereby in addition to the compounds of general formula I, there is obtained a compound of formula Ia,

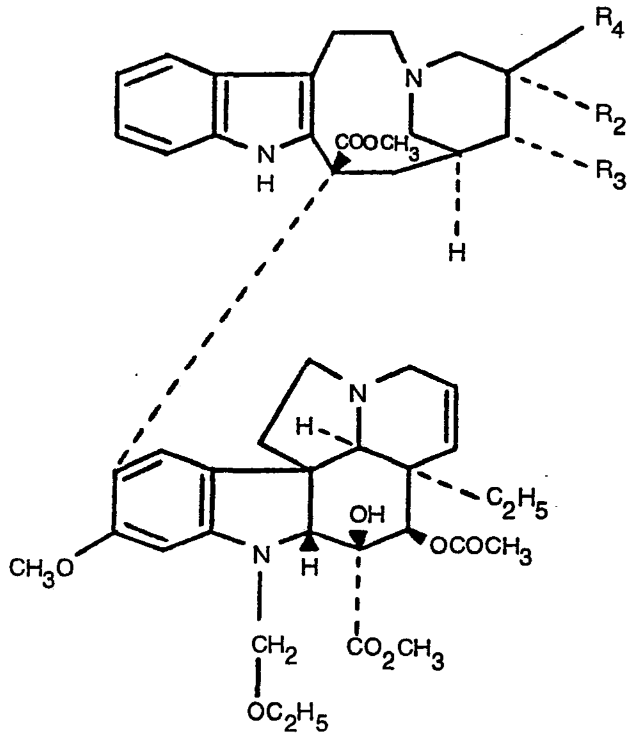

(Ia)

(wherein $R_2$, $R_3$ and $R_4$ are as hereinbefore defined). These derivatives of formula Ia are denoted herein as RGH derivatives. Thus, when vinblastine or an acid addition salt thereof is used as the starting material, in addition to N-desmethyl-vinblastine and vincristine, there is obtained a derivative of formula Ia denoted herein as RGH—4451. When leurosine or an acid addition salt thereof is used as the starting material, in addition to N-desmethyl-leurosine and N-desmethyl-N-formyl-leurosine, there is obtained a derivative of formula Ia denoted herein as RGH—4478. These new RGH derivatives may be characterised by their physical characteristics.

Thus the compound of formula Ia, denoted herein RGH—4451 has a melting point of 235°C to 238°C, a specific rotation of $[\alpha]_D^{25} = +30.5°$ (c = 1, chloroform), an infrared spectrum as illustrated in Fig. 1, a $^{1}H$—NMR spectrum as illustrated in Fig. 2 and a $^{13}C$—NMR spectrum as illustrated in Fig. 3. The compound RGH—4451 has a molecular weight of 854.449 corresponding to the formula $C_{48}$ $H_{62}$ $N_4$ $O_{10}$.

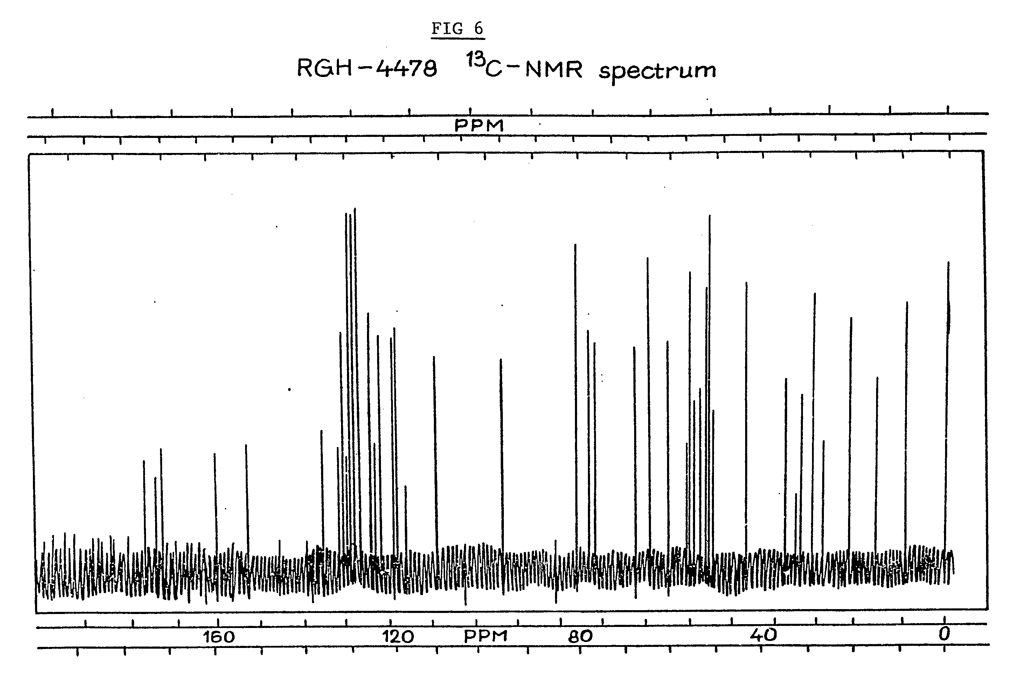

The compound of formula Ia, denoted herein RGH—4478 has a melting point of 180°C to 182°C, a specific rotation of $[\alpha]_D^{25} = +59.25°$ (c = 1, chloroform), an infrared spectrum as illustrated in Fig. 4, a $^{a}H$—NMR spectrum as illustrated in Fig. 5 and a $^{13}C$—NMR spectrum as illustrated in Fig. 6. The compound RGH—4478 has a molecular weight of about 852 corresponding to the formula $C_{48}$ $H_{60}$ $N_4$ $O_{10}$. It will be noted that the new RGH derivatives contain an N—$CH_2$—O—$C_2H_5$ group in place of the N-methyl group of the respective starting compounds in the vindoline moiety. The compound RGH—4451 and acid addition salts thereof form a further feature of the invention.

In addition to the new compounds, by the above oxidation in the presence of a small amount of ethanol, the corresponding N-desmethyl and N-desmethyl-N-formyl derivatives are also obtained. It should be noted that the presence of ethanol is not essential for the formation of these known compounds, which compounds are always obtained if the oxidation is carried out at a temperature of −30°C to −90°C. On the other hand the new RGH compounds are only obtained if a small amount of ethanol is present and if the temperature of oxidation exceeds −45°C the isolation of the new RGH compounds is rather complicated.

It has further been found that the new RGH compounds can be converted into the corresponding known N-desmethyl derivatives by acid treatment and the latter compounds can thereafter, if desired, be converted into the corresponding N-desmethyl-N-formyl derivatives by methods known *per se.*

The use of a different solvent system for the oxidation also has a technological advantage; due to the two-phase reaction system the oxidation, and the subsequent neutralisation, are less exothermic and thus easier to control and the danger of decomposition of the heat-sensitive diindole compounds is considerably decreased.

In the process according to the invention, as a starting material, technical or pharmacopeal vinblastine or a salt thereof, and leurosine or a salt thereof, may, for example be used. If an alkaloid of technical quality is employed, the product obtained can be purified for example, by chromatographic methods. If the starting material is of pharmacopeal quality, the product may be directly suitable for therapeutic application. As an oxidising agent chromic acid ($CrO_3$) or an alkali metal dichromate can be used.

Oxidation is carried out in the presence of a water-immiscible organic solvent in which the alkaloid starting materials and alkaloid products are readily soluble, acetic anhydride and optionally acetic acid, as well as ethanol. Typical representatives of such organic solvents are, for example, benzene and chlorinated hydrocarbons, preferably chloroform or methylene chloride.

Ethanol is used in an amount of from 0.1 to 10% by volume based on the volume of the water-immiscible organic solvent, preferably in an amount of 0.1 to 3.0%.

The temperature of the oxidation is from −30°C to −90°C. The N-desmethyl and N-desmethyl-N-formyl derivatives are always obtained under these conditions. If the oxidation is carried out at a temperature not exceeding −45°C also the RGH compounds can easily be isolated. If the reaction is performed over −45°C, N-desmethyl and N-formyl derivatives are easy to isolate but the isolation of the RGH compounds is cumbersome.

When the oxidation is complete, the pH of the reaction mixture is adjusted to 8 to 10. Care should be taken that the temperature does not exceed 50°C due to heat-sensitivity of these alkaloid products. The phases may then be allowed to separate and, if desired, the alkaloids may be isolated from the organic phase.

According to one embodiment of the invention the products are isolated from the reaction mixture after adjustment of the pH. In this case the alkaloid mixture obtained is separated by evaporating the organic phase and the various components, i.e. the N-desmethyl derivative, the N-desmethyl-N-formyl derivative and the RGH derivative may be separated, for example, by chromatography. Chromatography is conveniently performed on a column filled with partly deactivated alumina, elution being effected conveniently with various mixtures of benzene and a chlorinated hydrocarbon. The dientification of the alkaloids present in the various fractions may be effected by thin layer chromatography. The fractions containing the same alkaloids may be combined and the alkaloids then isolated therefrom for example by evaporation. The alkaloids obtained, if desired, may be purified and if desired converted into their acid addition salts e.g. physiologically compatible acid addition salts, preferably sulfate salts.

If desired, the N-desmethyl derivative may be formylated to give the corresponding N-desmethyl-N-formyl derivative. As a formylating agent a mixture of formic acid and acetic anhydride is preferably used. The formylation may, if desired, be effected on a mixture of the N-desmethyl and N-desmethyl-N-formyl derivatives or on the isolated N-desmethyl derivative.

According to a further embodiment of the process according to the invention the oxidation reaction mixture, after adjustment of the pH, is hydrolysed whereby the RGH derivative obtained is converted into the corresponding N-desmethyl derivative e.g. using dilute aqueous acid, for example a 0.5 to 5% aqueous sulphuric acid solution, hydrochloric acid solution or acetic acid. The obtained mixture of N-desmethyl and N-desmethyl-N-formyl derivatives may then be isolated and/or subjected to formylation as described above.

The process of the present invention is further illustrated by the following non-limiting Examples.

Example 1

5 g. (5.5 mmoles) of vinblastien sulfate are dissolved in a mixture of 1.2 l of a 1% solution of ethanol in chloroform and 250 ml. of acetic acid. The solution obtained is cooled to −55°C and a solution of 2.48 g. (24.48 mmoles) of chromium trioxide in 930 ml. of acetic anhydride at −55°C is added thereto. The resultant mixture is stirred for 40 minutes and then the pH is adjusted to 9 to 10 with a concentrated aqueous ammonium hydroxide solution having a temperature of −45°C to −50°C. Thereafter 5 l of water are added to the obtained two-phase reaction mixture and, after stirring for five minutes, the phases are allowed to separate. The aqueous phase is extracted with 0.5 l of chloroform. The chloroform phases are combined and treated with two 2.5 l portions of a 1% aqueous ammonium hydroxide solution. The phases are reseparated and the chloroform phase is dried over sodium sulphate and filtered. The filtrate is evaporated to dryness under reduced pressure.

4.0 g. of a mixture of vinblastien derivatives are obtained, which can be separated into its components by the following method:

The product obtained (4.0 g.) is dissolved in 24 ml. of a 2:1 mixture of benzene and chloroform. The solution obtained is then passed through a chromatographic column filled with 200 g. of alumina having an activity grade of III, which has previously been wetted with a 2:1 mixture of benzene and chloroform. The alkaloids are eluted by using the following eluants in the order given:

1.2 l of a 2:1 mixture of benzene and chloroform,
1.5 l of a 1:1 mixture of benzene and chloroform,
0.9 l of a 1:2 mixture of benzene and chloroform.

The various fractions obtained are examined by thin layer chromatography (t.l.c).

a) *Processing of the fraction obtained by elution with a 2:1 mixture of benzene and chloroform*

The first 400 ml. fraction of the eluate is alkaloid-free, the further eluates obtained with a 2:1 mixture of benzene and chloroform contain an alkaloid compound. By combining and evaporating the alkaloid-containing fractions 1.88 g of a crude new compound are obtained. The compound is designated as RGH—4451.

One or more recrystallisations of the crude product obtained from a two-fold amount of ethanol yields 1.30 g of crystalline, chromatographically uniform RGH—4451, having a melting point of 235°C to 238°C (decomp., after recrystallisation from ethanol twice).

$[\alpha]_D^{25} = +30.5°$ (c = 1, chloroform)

$R_f = 0.62$ (adsorbent: silica gel; eluting mixture: 100:5:5:5 mixture of diethyl ether, ethanol, benzene and diethylamine).

Mass spectrum: According to the mass spectrum the molecular weight of the compound is 854.449, which corresponds to the formula $C_{48}H_{62}N_4O_{10}$.

The mass number of this molecule is greater than that of vinblastine by 44, which corresponds to a $C_2H_4O$ unit. Fragmentation studies suggest that the said unit is linked to the vindoline part of the molecule.

The $^1H$—NMR spectrum is shown in Fig. 2. From this spectrum the N-methyl singlet present at 2.73 ppm in the spectrum of vinblastine is lacking. On the other hand at 4.48 ppm the multiplet peak of the methylene of the group N—$CH_2$—O can be seen, while the methyl triplet and methylene quartet of the group O—$C_2H_5$ appear at 1.05 and 3.35 ppm., respectively.

The $^{13}C$—NMR spectrum is shown in Fig. 3. The presence of the group N—$CH_2$—O is verified by the peak at 64.42 ppm. (which has a triplet multiplicity in the off-resonance spectrum).

On the basis of the above analysis an N—$CH_2O$—$C_2H_5$ group can be postulated in place of the N-methyl group of the vindoline moiety.

The IR spectrum of the compound can be seen in Fig. 1 where the characteristic group frequencies are as follows:

| | |
|---|---|
| 3470 $cm^{-1}$ | OH valence |
| 2970—2830 $cm^{-1}$ | OH valence vibrations |
| 1737 and 1230 $cm^{-1}$ | acetyl groups |
| 1612 $cm^{-1}$ | C=C bond |
| 1595 $cm^{-1}$ | aromatic skeleton |
| 742 $cm^{-1}$ | 4 AR—H next to each other. |

The characteristic new bands of RGH—4451 having a low intensity appear at 995, 1170 and 1360 $cm^{-1}$.

It can also be seen that the low-frequency bands at 1090 $cm^{-1}$ and 700 $cm^{-1}$ characteristic of vinblastine disappear.

b) *Processing of the fraction obtained by elution with 1:1 mixture of benzene and chloroform*

The first 1000—1200 ml. fraction of the eluate obtained when eluting with 1.5 l of a 1:1 mixture of benzene and chloroform is a so-called mixed fraction consisting of the RGH—4451 compound and N-desmethyl-vinblastine. The remaining 300 to 500 ml.-fraction contains N-desmethyl-vinblastine substantially free of RGH—4451.

The mixed fraction is evaporated to give 0.57 g. of a mixed solid, which is converted into pure N-desmethyl-vinblastine by the following technique: The evaporation residue is dissolved in 10 ml. of benzene, the benzene solution is extracted with three 15 ml. portions of a 2% aqueous hydrochloric acid solution, the phases are separated and the pH of the aqueous phase is then adjusted to 9 with a concentrated aqueous ammonium hydroxide solution. The obtained alkaline solution is extracted with three 10 ml. portions of chloroform and the chloroform extract is evaporated. 0.56 g of N-desmethyl-vinblastine are obtained.

By evaporating the N-desmethyl-vinblastine fractions 0.08 g. of N-desmethyl-vinblastine are obtained, which is combined with the N-desmethyl-vinblastine obtained from the mixed fraction. N-desmethyl-vinblastine is converted into vincristine as described in Hungarian Patent Specification No. 165,599.

Yield: 0.63 g. of vincristine.

Melting point: 218°C to 220°C.

c) *Processing of the fraction obtained by elution with a 1:2 mixture of benzene and chloroform*

From the fraction obtained by elution with 0.9 l of a 1:2 mixture of benzene and chloroform, vincristine can be isolated by evaporation.

Yield: 1.22 g. of vincristine

Melting point: 220°C.

Example 2

5 g. (5.5 mmoles) of vinblastine sulfate are dissolved in a mixture of 1.2 l of a 1% solution of ethanol in chloroform and 250 ml. of acetic acid. The solution obtained is cooled to −5°C and a solution of 2.48 g. (24.48 mmoles) of chromium trioxide in 930 ml. of acetic anhydride at −5°C is added thereto. The resultant mixture is stirred for 40 minutes and then the pH is adjusted to 9 to 10 with a concentrated aqueous ammonium hydroxide solution cooled to −45°C to −50°C. Thereafter 5 l of water are added to the two-phase reaction mixture and, after stirring for five minutes, the phases are allowed to separate. The aqueous phase is extracted with 0.5 l of chloroform. The chloroform phases are combined and treated with two 2.5 l portions of a 1% aqueous ammonium hydroxide solution. The phases are reseparated and the chloroform phase is extracted with three 0.3 l portions of an aqueous sulfuric acid solution, then the pH of the extract is adjusted to 9 with a concentrated aqueous ammonium hydroxide solution. The alkaline extract is extracted with three 0.2 l portions of chloroform. Evaporation of the extract affords an evaporation residue consisting of N-desmethyl-vinblastine and vincristine, which is converted to vincristine by formylation carried out as described in Hungarian Patent Specification No. 165,599. By treatment with a solution of sulfuric acid in ethanol, the product obtained can be converted into vincristine sulfate.
Yield: 3.6 g of vincristine sulfate.

Example 3

5 g. (5.5 mmoles) of vinblastine sulfate are dissolved in a mixture of 1.2 l of a 1% solution of ethanol in chloroform and 250 ml. of acetic acid. The solution obtained is cooled to −55°C and a solution of 2.48 g. (24.48 mmoles) of chromium trioxide in 930 ml. of acetic anhydride at −55°C is added thereto. The resultant mixture is stirred for 40 minutes and then poured into a mixture of 6 l of water and 4 l of a concentrated aqueous ammonium hydroxide solution cooled to +2°C to +5°C. The resultant mixture is stirred for 5 minutes at a temperature below 20°C, ensuring that the pH of the aqueous phase remains at least 8. The phases are then separated and the aqueous phase is extracted with 0.5 l of chloroform. The extract is combined with the chloroform phase. The resultant chloroform solution is extracted with two 2.5 l portions of a 1% aqueous ammonium hydroxide solution, the phases are separated and the chloroform phase is dried. It is then either

a) evaporated according to Example 1, followed by separation of the diindole-alkaloid mixture obtained weighing 4 g. into its components, or

b) is converted into vincristine by an acid treatment and a subsequent formyulation as described in Example 2.

If the a) reaction route is followed the yield is identical with that obtained in Example 1, while the b) reaction route affords the same yield as Example 2.

Example 4

5 g. (5.5 mmoles) of a vinblastine sulfate are dissolved in a mixture of 1.2 l of a 0.5% solution of ethanol in methylene chloride and 250 ml. of acetic acid. The solution obtained is cooled to −55°C and a solution of 2.48 g. (24.48 mmoles) of chromium trioxide in 930 ml. of acetic anhydride at −55°C is added thereto. The resultant mixture is stirred for 40 minutes and then the pH is adjusted to 9 to 10 with a concentrated aqueous ammonium hydroxide solution cooled to −45°C to −50°C. Thereafter 5 l of water are added to the two-phase reaction mixture and, after stirring for five minutes, the phases are allowed to separate. The aqueous phase is extracted with 0.5 l of methylene chloride. The methylene chloride phases are combined and treated with two 2.5 l portions of a 1% aqueous ammonium hydroxide solution. The phases are reseparated and the methylene chloride phase is dried with sodium sulfate and filtered. The filtrate is evaporated to dryness under reduced pressure.

Yield: 3.5 g of a mixture of diindole-alkaloids, which is separated into its components following the method described in Example 1.

The physical characteristics of RGH— 4451 and vincristine, are identical with the corresponding characteristics of the products of Example 1.

Example 5

5 g. of leurosine sulfate are dissolved in a mixture of 1.2 l of a 1% solution of ethanol in chloroform and 250 ml of acetic acid. The solution obtained is cooled to −55°C and a solution of 2.48 g. (24.48 mmoles) of chromium trioxide in 930 ml. of acetic anhydride at −55°C is added thereto. The resultant mixture is stirred for 40 minutes and then the pH is adjusted to 9 to 10 with a concentrated aqueous ammonium hydroxide solution having a temperature of −45°C to −50°C. Thereafter 5 l of water are added to the two-phase reaction mixture and after stirring for five minutes the phases are allowed to separate. The chloroform phase is set aside and the aqueous phase is extracted with 0.5 l of chloroform. The chloroform phases are combined and extracted with two 2.5 l portions of a 1% aqueous ammonium hydroxide solution. The phases are re-separated and the chloroform phase is dried with sodium sulfate then filtered. The filtrate is evaporated to dryness under reduced pressure.

Yield: 4.5 g. of a mixture of leurosine derivatives, which is separated into the corresponding components by the following method:

The product obtained (4.5 g.) is dissolved in 27 ml. of a 3:1 mixture of benzene and chloroform and the solution obtained is chromatographed on a column filled with alumina having an activity grade of III and treated with a 3:1 mixture of benzene and chloroform. The alkaloids are eluted with the following eluants in the order given:

3.5 l of a 3:1 mixture of benzene and chloroform,
1 l of a 2:1 mixture of benzene and chloroform,
2.5 l of a 1:2 mixture of benzene and chloroform.

250 ml. fractions are collected and the alkaloids contained therein are examined by thin layer chromatography (t.l.c.).

The 1st to 6th fractions (1.5 l) are alkaloid-free, the 7th to 14th fractions (3:1 mixture of benzene and chloroform) and the 15th to 17th fractions (2:1 mixture of benzene and chloroform) contain the new compound identified as RGH—4478. The 18th fraction obtained with a 2:1 mixture of benzene and chloroform and the 19th to 23rd fractions obtained with a 1:2 mixture of benzene and chloroform contain N-desmethyl-leurosine, while the 24th to 28th fractions obtained by elution with a 1:2 mixture of benzene and chloroform contain N-desmethyl-N-formyl-leurosine.

The eluate fractions are processed as follows:

a) the 7th to 17th fractions are combined and evaporated. 2.13 g. of crude RGH—4478 are obtained, which is purified by crystallisation from 5 ml. of ethanol at 5°C. If desired, the product is dissolved in chloroform, the solution obtained is evaporated and crystallisation from ethanol is repeated.

Yield: 1.6 g. of RGH—4478

Melting point: (after recrystallisation from ethanol twice): 180°C to 182°C.

$[\alpha]_D^{25} = +59.25°$ (c = 1, chloroform).

$R_f = 0.37$ (adsorbent: silica gel; eluting mixture: a 100:5:5:5 mixture of diethyl ether, ethanol, benzene and diethylamine).

Molecular weight: 852.

The $^1H$—NMR spectrum is shown in Fig. 4. From this spectrum the N-methyl singlet characteristic of the starting material is lacking. On the other hand at 4.48 ppm. the multiplet peak of the methylene of the group —N—$CH_2$—O can be seen, while the methyl triplet and methylene quartet of the group O—$C_2H_5$ appear at 1.10 and 3.38 ppm., respectively.

The $^{13}C$—NMR spectrum is shown in Fig. 5, while the IR spectrum is shown in Fig. 6.

The characteristic group frequencies of the IR spectrum of the product are as follows:

| | |
|---|---|
| 3470 $cm^{-1}$ | OH valence |
| 2970—2830 $cm^{-1}$ | OH valence vibrations |
| 1737 and 1230 $cm^{-1}$ | acetyl groups |
| 1612 $cm^{-1}$ | C=C bond |
| 1595 $cm^{-1}$ | aromatic skeleton |
| 742 $cm^{-1}$ | 4 AR—H next to each other |
| 823 $cm^{-1}$ | epoxide ring |

b) The 18th to 23rd fractions are combined. The RGH—4478 traces are eliminated by extraction with a 2% sulfuric acid solution and adjustment of the pH of the acid aqueous phase to 9 with a concentrated aqueous ammonium hydroxide solution. The solution obtained is then extracted with three 0.2 l portions of chloroform and the chloroform extract is evaporated.

Yield: 0.25 g. of N-desmethyl-leurosine.

The product obtained, if desired, is converted into N-desmethyl-N-formyl-leurosine by formylation carried out as described in Hungarian Patent Specification No. 165,986.

Yield: 0.14 g of N-desmethyl-N-formyl-leurosine

c) The 24th to 28th fractions are combined and evaporated.

Yield: 4.42 g. of N-desmethyl-N-formyl-leurosine.

## Example 6

Following the procedure described in Example 2 but starting with 5 g. of leurosine sulfate instead of vinblastine sulfate, 3.7 g. of N-desmethyl-N-formyl-leurosine sulfate are obtained.

Example 7

5 g. (5.5 mmoles) of vinblastine sulfate, dried until alcohol-free, are dissolved in 1000 ml. of ethanol-free methylene chloride, and to the solution obtained are added 120 ml. of acetic acid and 3.2 ml. (10 equivalent) of ethanol. The resultant solution is cooled to −55°C and a solution of 2.5 g. of chromium trioxide in 470 ml. of acetic anhydride cooled to −55°C is added thereto. The resultant mixture is stirred at −55°C for 40 to 45 minutes and then the pH is adjusted and the methylene chloride phases are evaporated as described in Example 4.

Yield: 4 g. of a diindole-alkaloid mixture.

The 4 g. of mixed product obtained are dissolved in a 8:2 mixture of benzene and chloroform and the solution obtained is passed through a chromatographic column filled with 200 g. of alumina, having an activity grade of III. Elution is carried out in the following way:

As an eluant initially is used 5 to 6 l of a 8:2 mixture of benzene and chloroform. The eluates are combined and evaporated. 2.4 g. of crude RGH—4451 are obtained. Recrystallisation of the crude product from ethanol affords crystalline RGH—4451, which has the same characteristics as described in Example 1.

Elution is then repeated with 3 to 4 l of a 1:1 mixture of benzene and chloroform. The combined fractions containing N-desmethyl-vinblastine as well as the fractions containing vincristine are combined and evaporated separately.

Yield: 0.4 g. of N-desmethyl-vinblastine and 0.8 g. of vincristine.

Example 8

Following the procedure described in Example 7 but carrying out the reaction in the presence of 9.5 ml. (30 equivalent) of ethanol, the products of Example 7 are obtained, with the same yields. The physical characteristics of the products are also identical with those described in Example 7.

As mentioned above the new compounds RGH—4451 and RGH—4478 as well as their physiologically compatible acid addition salts possess interesting pharmacological properties and, in particular, may be used as antitumour agents. Although it will be appreciated that, for pharmaceutical use the acid addition salts of the compounds RGH—4451 and RGH—4478 must be physiologically compatible, other acid addition salts may find use, for example, in the preparation of the compounds RGH—4451 and RGH—4478 and their physiologically compatible acid addition salts.

From pharmacological tests we have carried out we have concluded that from a pharmacological point of view RGH—4451 is the most advantageous amount the compounds obtained.

The acute toxicity of RGH—4451 was tested on mice. The $LD_{50}$ value of this new compound amounted to 70 mg/kg i.p., which is 17 times that of vincristine, about 10 times higher than that of vinblastine and 2.5 times higher than that of N-desmethyl-N-formyl-leurosine. The $LD_{50}$ value of RGH—4478, as measured on mice is greater than 70 mg/kg i.p.

The lethal dose killed the animals on the 2nd to 5th day. The deaths were not accompanied by paralytic symptoms and, in contrast with the results obtained with vincristine, the surviving animals were not crippled even temporarily.

The inhibition of tumour growth was initially studied in a tissue culture. A 20 mg./ml. solution of RGH—4451 and of RGH—4478 in Fischer medium was prepared and subsequently diluted to the desired concentration. The number of cells in P 388 cultures was determined 24 and 48 hours after treatment.

In a concentration of $10^{-1}$ ug/ml. RGH—4451 considerably inhibited the growth of P 388 tumour cells. The inhibition was 43% after 24 hours and 89% after 48 hours. In *in vitro* tests this compound was 100 times less toxic than vincristine and 4 times less toxic than N-desmethyl-N-formyl-leurosine.

The inhibition of tumour growth was examined also on transplanted rodent tumours.

*Effect on P 388 leukemia tumours of mice*

$10^6$ P 388 leukemia cells were transplanted into $BDF_1$ hybrid mice intraperioneally. Administration of RGH—4451 was started the day following the transplantation. The results obtained are summarised in the following table:

| Dose mg./kg. i.p. | Number of treatments | Schedule | Mean duration of life: expressed in days, treated | control | expressed in % of treated/control |
|---|---|---|---|---|---|
| RGH—4451 | | | | | |
| 3.2 | 9 | every day | 22.3 | 12.3 | 181 |
| 5.0 | 9 | every day | 21.5 | 13.2 | 163 |
| 6.4 | 9 | every day | 20.5 | 12.3 | 166 |
| 10 | 5 | every second day | 21.8 | 10.5 | 208 |
| 20 | 6 | every day | 8.5 | 13.2 | 64 |
| 20 | 1 | — | 19.4 | 10.5 | 185 |
| RGH—4478 | | | | | |
| 2 | 9 | every day | 10.2 | 9.6 | 106 |
| 4 | 9 | every day | 11.2 | 9.6 | 116 |

From the table it can be seen that the duration of life of the tumourous animals has considerably been prolonged by administration of 3.2 to 6.4 mg-kg. doses every day. A 10 mg./kg. dose administered every second day had an even more favourable effect. It can also be seen that a single 20 mg.kg. dose is also effective, but this large dose cannot be administered every day due to its toxicity. A similar effect was achieved with 0.1 to 0.3 mg./kg./day doses of vincristine, 1.0 to 4.0 mg./kg./day doses of N-desmethyl-N-formyl-leurosine and 0.1 to 0.4 mg./kg./day doses of vinblastine.

*Effect on Ehrlich ascites carcinoma*

Transplantation was carried out into outbred Swiss mice at a level of 5 × $10^6$ ascites tumour cells.

Administration of RGH—4451 was started 24 hours after transplantation. The results obtained are shown in the following table.

| Dose mg./kg. i.p. | Number of treatments | Schedule | Mean duration of life: expressed in days, treated | control | expressed in % of treated/control |
|---|---|---|---|---|---|
| 1.0 | 9 | every day | 54.2 | 15.4 | 341 |
| 2.0 | 9 | every day | 43.00 | 16.4 | 261 |
| 4.0 | 9 | every day | 36.4 | 16.4 | 222 |
| 6.0 | 9 | every day | 48.2 | 15.9 | 303 |

From the above results it can be clearly seen that the effect of doses between 1.0 and 6.0 mg./kg. is significant.

*Effect on Nk-Ly ascites lymphoma*

Tests were carried out analogously with the Ehrlich ascites test.

The results obtained are listed in the following table.

| Dose mg./kg. i.p. | Number of treatments | Schedule | Mean duration of life: expressed in days treated/control | expressed in % of treated*/control | On the 65th day: alive | tumour-free |
|---|---|---|---|---|---|---|
| 2.0 | 9 | every day | 64.1/16.4 | 392 | 9/10 | 6/10 |
| 4.0 | 9 | every day | 62.9/16.4 | 384 | 8/10 | 7/10 |
| 6.0 | 9 | every day | 65.8/16.7 | 394 | 8/10 | 4/10 |
| 20 | 1 | — | 65.5/16.7 | 392 | 8/10 | 3/10 |

* i.e. staying alive for four times the mean duration of life of the control group. The maximum is therefore 400%

From the results it can be seen that 2 to 6 mg./kg. doses administered once a day have a remarkable antitumour effect, a major part of the treated animals are tumour-free on the 65th day. A similar result is obtained when a single large dose is administered.

0.05 to 0.25 mg./kg. daily doses of vincristine and 1 to 4 mg./kg. daily doses of N-desmethyl-N-formyl-leurosine have a similar effect. In this case, however, the administration of a large single dose did not increase the duration of life.

Effect on S 180 sc. carcinoma

S 180 sc. tumour tissues were transplanted into Swiss mice subcutaneously. Treatment was started 24 hours after transplantation. RGH—4451 was administered daily in a dose of 2, 4, 8 and 16 mg./kg. for 8 days, respectively i.p. The tumour growth inhibition was evaluated on the 11th day on the basis of the weight of tumours. The change in weight of the spleen was also monitored.

The tumour growth inhibition observed upon administration of RGH—4451 was about the same as in the case of known diindole-alkaloids (34 to 56% depending on the dose) but the daily dose of the new compound could be higher and there was no substantial decrease in the weight of spleen even at as high doses as 16 mg./kh.

*Effect of Guerin sc. carcinoma*

Tumour was transplanted into Wistar rats subcutaneously. Administration of RGH—4451 was started on the day following the transplantation and the compound was administered on six subsequent days intraperitoneally. The inhibition of tumour growth was evaluated on the 22nd day. The results obtained are listed in the following table.

| Dose mg./kg. i.p. | Weight of tumour: Treated | Control | Inhibition | Mortality until the 22nd day/ number of animals |
|---|---|---|---|---|
| 2.0 | 10.7 | 18.9 | 43 | 0/6 |
| 6.0 | 8.69 | 44.9 | 81 | 0/6 |
| 10.0 | 14.64 | 44.9 | 68 | 0/6 |
| 14.0 | — | — | — | 5/6 |

2 to 10 mg./kg. i.p. doses of RGH—4451 strongly inhibited the growth of Guerin carcinoma.

From the tests described hereinabove it can be concluded that 1.0 to 10.0 mg./kg. daily doses or 20 mg./kg. single doses of RGH—4451 have a significant tumour growth inhibition effect on rodent tumours and prolong the duration of life.

In contrast with vincristine, paralytic symptoms are entirely lacking even when sublethal doses are administered. The therapeutic width of the tested new compound is greater than that of vincristine and N-desmethyl-N-formyl-leurosine as shown in the following table.

| | MTD/min eff. D |
|---|---|
| RGH—4451 | 10 |
| Vincistine | 6 |
| N-desmethyl-N-formyl-leurosine | 4 |

Thus in human therapy for infusion or intravenous administration 0.5 to 1.0 mg.kg. daily doses may advantageously be used.

According to a still further feature of the present invention there are provided pharmaceutical compositions comprising, as active ingredient, the compound RGH—4451 as defined herein, or a physiologically compatible acid addition salt thereof, in association with a pharmaceutical carrier or excipient.

For pharmaceutical administration the compound RGH—4451 and its physiologically compatible acid addition salts may be incorporated into conventional preparations, preferably in a form suitable for parenteral administration. Thus, for example, the active ingredient may be lyophilised and procesed into dry ampoules which, in use, are mixed with the contents of a solvent ampoule, preferably immediately prior to administration. The solvent ampoule will contain a carrier, preferably distilled water or a physiological sodium chloride solution, optionally together with a preserving agent (e.g. benzyl alcohol), an antioxidant, milk sugar or a buffer.

Administration is carried out preferably by infusion of a solution of the active ingredient in a physiological saline solution.

The compositions according to the invention may, if desired, contain other pharmaceutically active compounds e.g. an adjuvant (analgesic, cardiac restorative) or a known cytostatic. Advantageously the compositions may be formulated as dosage units, each unit being adapted to supply a fixed dose of active ingredient.

**Claims for the contracting states: BE CH DE FR GB IT LU NL SE**

1. A process for the preparation of alkaloids of the formula I

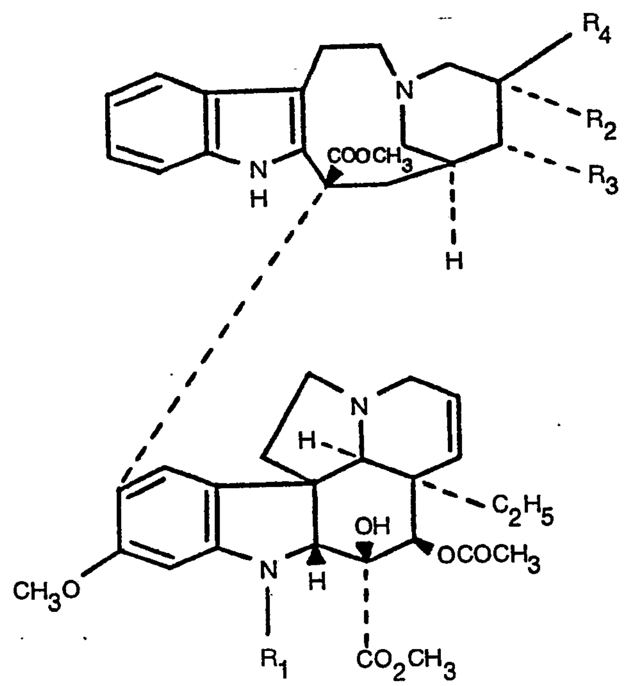

(I)

(wherein $R_1$ represents a hydrogen atom or a formyl group and *either* $R_2$ represents an ethyl group, $R_3$ represents a hydrogen atom and $R_4$ represents a hydroxy group *or* $R_2$ and $R_3$ together represent an epoxy group and $R_4$ represents an ethyl group) and acid addition salts thereof which comprises reacting an alkaloid of formula II,

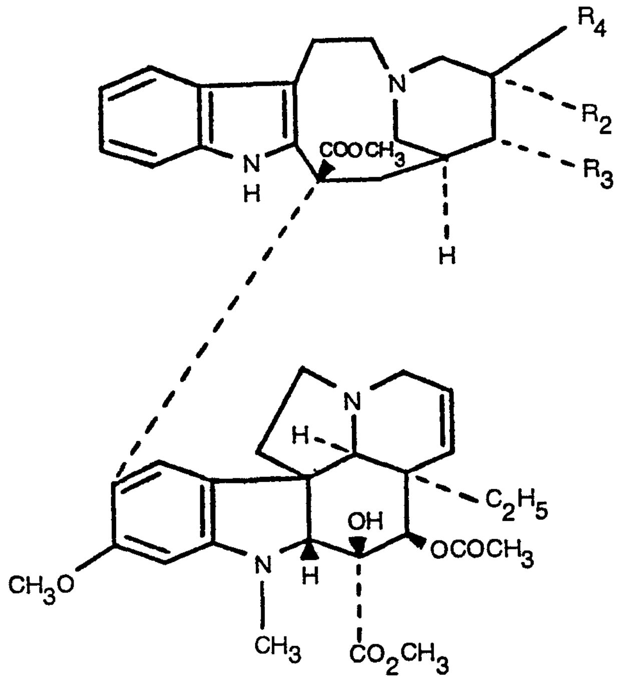

(II)

(wherein $R_2$, $R_3$ and $R_4$ are as defined above) or an acid addition salt thereof with chromic acid or an alkali metal dichromate, at a temperature of from −30°C to −90°C in the presence of a water-immiscible organic solvent in which the alkaloid starting materials and alkaloid products are readily soluble, acetic anhydride and optionally glacial acetic acid, the reaction medium also cotaining ethanol in an amount of from 0.1 to 10% by volume based on the volume of the water-immiscible organic solvent; thereafter adjusting the pH of the reaction mixture to from 8 to 10; and isolating the products obtained and/or subjecting the said products to further treatment; whereby in addition to the compounds of general formula I, a compound of formula Ia,

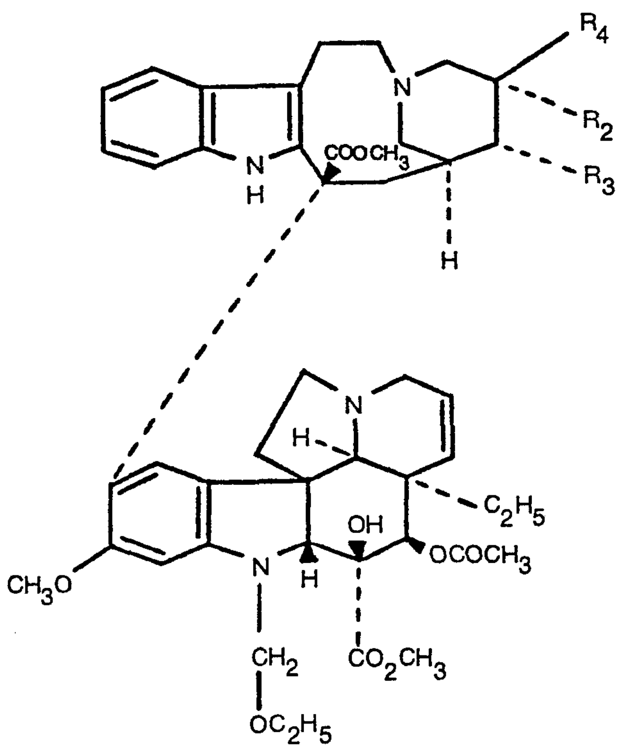

(Ia)

(wherein $R_2$, $R_3$ and $R_4$ are as defined above) or an acid addition salt thereof is obtained.

2. A process as claimed in claim 1 wherein the water-immiscible organic solvent is chloroform or methylene chloride.

3. A process as claimed in claim 1 or claim 2 wherein the oxidation is effected in a medium containing ethanol in an amount of from 0.1 to 3.0% by volume based on the volume of the water-immiscible organic solvent.

4. A process as claimed in any one of the preceding claims wherein the oxidation reaction is effected at a temperature of from −45°C to −90°C.

5. A process as claimed in any one of the preceding claims wherein there are isolated from the reaction mixture after adjustment of the pH, either

a) where vinblastine or an acid addition salt thereof is used as the starting material: N-desmethyl-vinblastine, vincristine and a compound of formula Ia as defined in claim 1, in which $R_2$ represents an ethyl group, $R_3$ represents a hydrogen atom and $R_4$ represents a hydroxy group, denoted herein RGH—4451, the said compound RGH—4451 having a melting point of from 235°C to 238°C and a specific rotation of $[\alpha]_D^{25} = +30.5$ (c = 1, chloroform), or

b) where leurosine or an acid addition salt thereof is used as the starting material: N-desmethyl-leurosine, N-desmethyl-N-formyl-leurosine and a compound of formula Ia as defined in claim 1, wherein $R_2$ and $R_3$ together represent an epoxy group and $R_4$ represents an ethyl group, denoted herein RGH—4478, the said compound RGH—4478 having a melting point of from 180°C to 182°C and a specific rotation of $[\alpha]_D^{25} = +59.25°$ (c = 1, chloroform),

which products are optionally subsequently converted into their acid addition salts.

6. A process as claimed in any one of the preceding claims wherein the reaction mixture, after adjustment of the pH, is hydrolysed and the mixture of N-desmethyl and N-desmethyl-N-formyl derivatives obtained is either separated or subjected to formylation whereby the N-desmethyl derivative is converted to the N-desmethyl-N-formyl derivative.

7. A compound of formula Ia as defined in claim 1 wherein $R_2$ represents an ethyl group, $R_3$ represents a hydrogen atom and $R_4$ represents a hydroxy group, said compound having a melting point of 235°C to 238°C and a specific rotation of $[\alpha]_D^{25} = +30.5°$ (c = 1, chloroform) and acid addition salts thereof.

8. Pharmaceutical compositions comprising, as active ingredient, a compound of formula Ia as claimed in claim 7 or a physiologically compatible acid addition salt thereof in association with a pharmaceutical carrier or excipient.

9. A compound as claimed in claim 7 for use as an anti-tumour agent.

**Claims for the contracting state AT**

1. A process for the preparation of alkaloids of the formula I

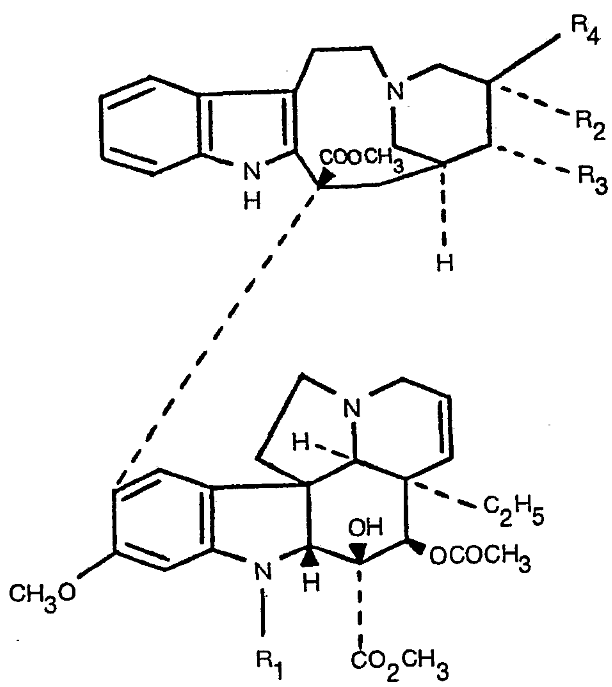

(I)

(wherein $R_1$ represents a hydrogen atom or a formyl group and *either* $R_2$ represents an ethyl group, $R_3$ represents a hydrogen atom and $R_4$ represents a hydroxy group *or* $R_2$ and $R_3$ together represent an epoxy group and $R_4$ represents an ethyl group) and acid addition salts thereof which comprises reacting an alkaloid of formula II,

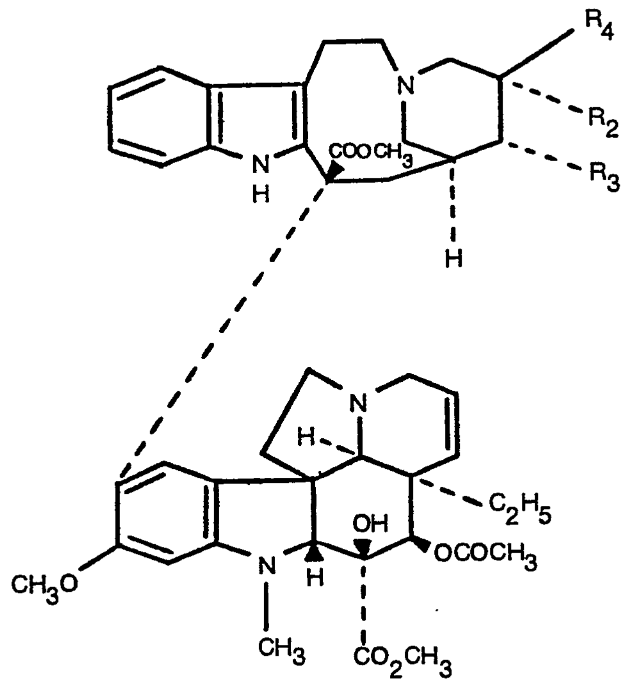

(II)

(wherein $R_2$, $R_3$ and $R_4$ are as defined above) or an acid addition salt thereof with chromic acid or an alkali metal dichromate, at a temperature of from −30°C to −90°C in the presence of a water-immiscible organic solvent in which the alkaloid starting materials and alkaloid products are readily soluble, acetic anhydride and optionally glacial acetic acid, the reaction medium also cotaining ethanol in an amount of from 0.1 to 10% by volume based on the volume of the water-immiscible organic solvent; thereafter adjusting the pH of the reaction mixture to from 8 to 10; and isolating the products obtained and/or subjecting the said products to further treatment; whereby in addition to the compounds of general formula I, a compound of formula Ia,

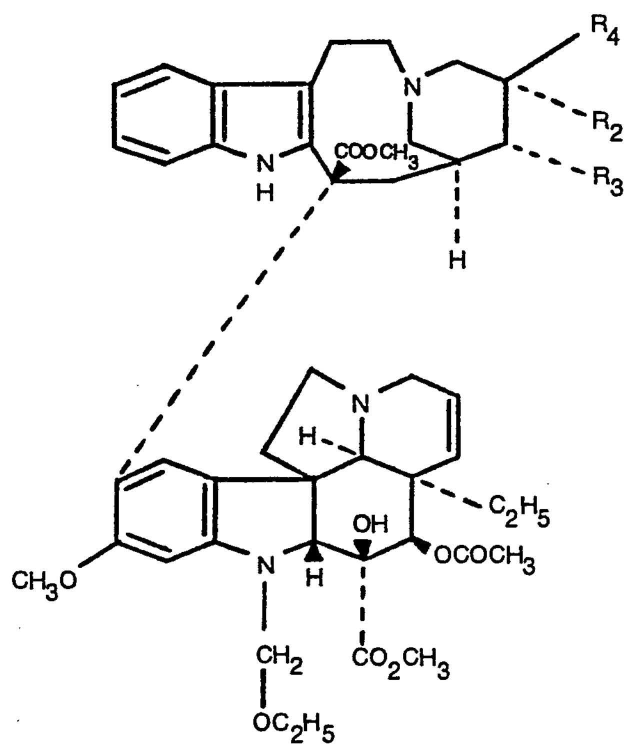

(Ia)

(wherein $R_2$, $R_3$ and $R_4$ are as defined above) or an acid addition salt thereof is obtained.

2. A process as claimed in claim 1 wherein the water-immiscible organic solvent is chloroform or methylene chloride.

3. A process as claimed in claim 1 or claim 2 wherein the oxidation is effected in a medium containing ethanol in an amount of from 0.1 to 3.0% by volume based on the volume of the water-immiscible organic solvent.

4. A process as claimed in any one of the preceding claims wherein the oxidation reaction is effected at a temperature of from −45°C to −90°C.

5. A process as claimed in any one of the preceding claims wherein there are isolated from the reaction mixture after adjustment of the pH, either

a) where vinblastine or an acid addition salt thereof is used as the starting material: N-desmethyl-vinblastine, vincristine and a compound of formula Ia as defined in claim 1, in which $R_2$ represents an ethyl group, $R_3$ represents a hydrogen atom and $R_4$ represents a hydroxy group, denoted herein RGH—4451, the said compound RGH—4451 having a melting point of from 235°C to 238°C and a specific rotation of $[\alpha]_D^{25} = +30.5$ (c = 1, chloroform), or

b) where leurosine or an acid addition salt thereof is used as the starting material: N-desmethyl-leurosine, N-desmethyl-N-formyl-leurosine and a compound of formula Ia as defined in claim 1, wherein $R_2$ and $R_3$ together represent an epoxy group and $R_4$ represents an ethyl group, denoted herein RGH—4478, the said compound RGH—4478 having a melting point of from 180°C to 182°C and a specific rotation of $[\alpha]_D^{25} = +59.25°$ (c = 1, chloroform),

which products are optionally subsequently converted into their acid addition salts.

6. A process as claimed in any one of the preceding claims wherein the reaction mixture, after adjustment of the pH, is hydrolysed and the mixture of N-desmethyl and N-desmethyl-N-formyl derivatives obtained is either separated or subjected to formylation whereby the N-desmethyl derivative is converted to the N-desmethyl-N-formyl derivative.

7. A process as claimed in claim 6 wherein hydrolysis is effected by means of 0.5 to 5% aqueous sulphuric acid, hydrochloric acid or acetic acid.

8. A process as claimed in either of claims 6 and 7 wherein formylation is effected by means of a mixture of formic acid and acetic anhydride.

**Revendications pour les Etats contractants: BE CH DE FR GB IT LI LU NL SE**

1. Procédé pour la préparation d'alcaloïdes de formule I

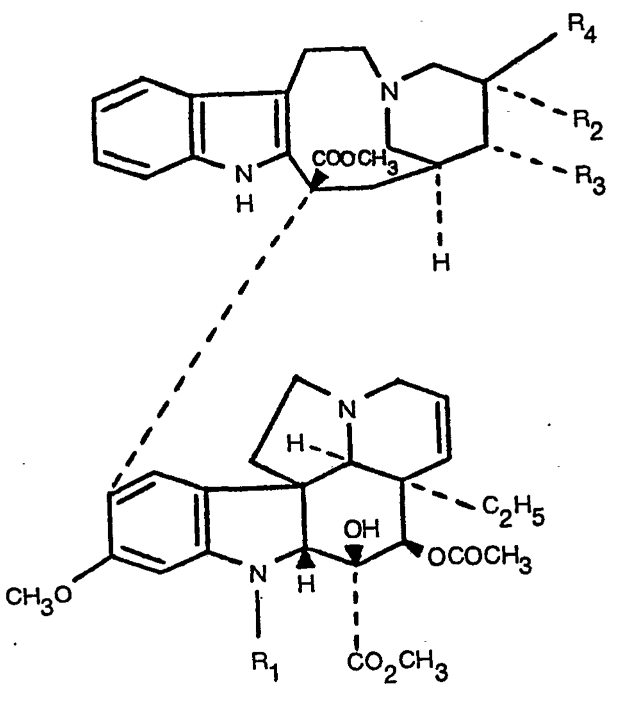

(I)

(dans laquelle $R_1$ représente un atome d'hydrogène ou un groupe formyle, et soit $R_2$ représente un groupe éthyle, $R_3$ représente un atome d'hydrogène et $R_4$ représente un groupe hydroxyle, soit $R_2$ et $R_3$ représentent ensemble un groupe époxy et $R_4$ représente un groupe éthyle) et leurs sels d'addition acides, qui consiste à faire réagir un alcaloïde de formule II

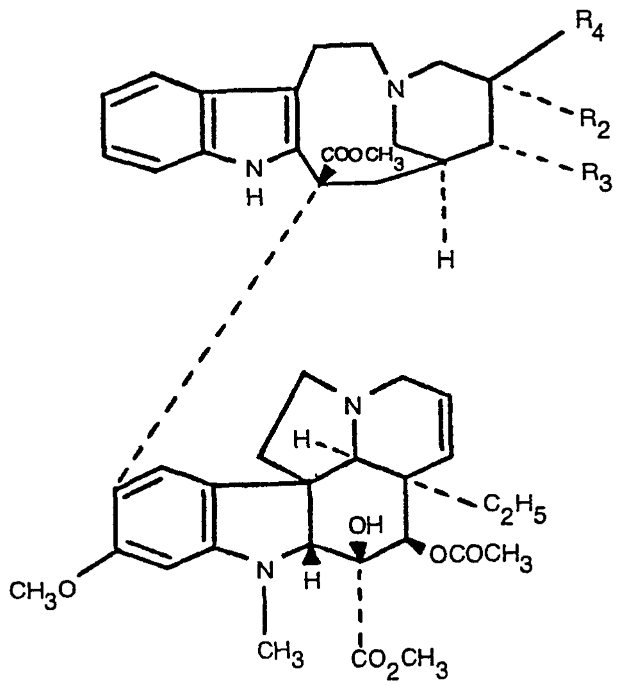

(II)

(dans laquelle $R_2$, $R_3$ et $R_4$ ont les définitions ci-dessus) ou un de ses sels d'addition acides avec l'acide chromique ou un bichromate de métal alcalin, à une température allant de −30°C à −90°C en présence d'un solvant organique non miscible avec l'eau dans lequel les matières de départ alcaloïdes et les produits alcaloïdes sont facilement solubles, d'anhydride acétique et éventuellement d'acide acétique glacial, le milieu réactionnel contenant également de l'éthanol à raison de 0,1 à 10% en volume, rapporté au volume du solvant organique non miscible avec l'eau; ensuite à régler le pH du mélange réactionnel entre 8 et 10; et à isoler les produits obtenus et/ou à soumettre lesdits produits à un traitement ultérieur; ce qui fait qu'en plus des composés de formule générale I, on obtient un composé de formule Ia

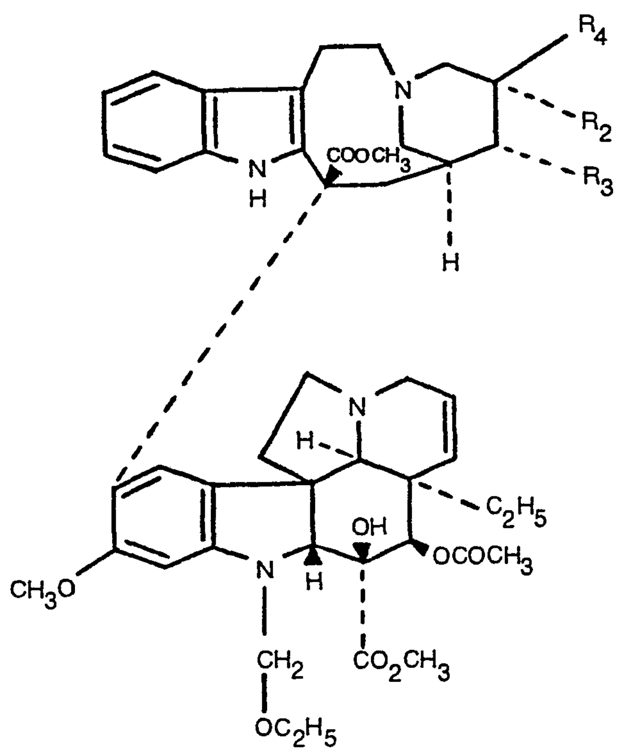

(Ia)

(dans laquelle $R_2$, $R_3$ et $R_4$ ont les définitions ci-dessus) ou bien un sel d'addition acide de ce composé.

2. Procédé tel que revendiqué dans la revendication 1, dans lequel le solvant organique non miscible avec l'eau est le chloroforme ou le chlorure de méthylène.

3. Procédé tel que revendiqué dans les revendications 1 ou 2, dans lequel l'oxydation est effectuée dans un milieu contenant de l'éthanolen une quantité allant de 0,1 à 3% en volume, rapporté au volume du solvant organique non miscible avec l'eau.

4. Procédé tel que revendiqué dans l'une quelconque des revendications précédentes, dans lequel la réaction d'oxydation est effectuée à une température allant de —45°C à —90°C.

5. Procédé tel que revendiqué dans l'une quelconque des revendications précédentes, dans lequel on isole du mélange réactionnel après réglage du pH, soit

a) si la vinblastine ou un sel d'addition acide de celle-ci est utilisé(e) comme matière de départ: la N-desméthyl-vinblastine, la vincristine et un composé de formule Ia tel que défini dans la revendication 1, dans lequel $R_2$ représente un groupe éthyle, $R_3$ représente un atome d'hydrogène et $R_4$ représente un groupe hydroxyle, appelé ici RGH—4451, ledit composé RGH—4451 ayant un point de fusion compris entre 235°C et 238°C, et un pouvoir rotatoire spécifique de $[\alpha]_D^{25} = +30{,}5°$ (c = 1, chloroforme), soit

b) si la leurosine ou un sel d'addition acide de celle-ci est utilisé(e) comme matière de départ: la N-desméthyl-leurosine, la N-desméthyl-N-formyl-leurosine et un composé de formule Ia tel que défini dans la revendication 1, dans lequel $R_2$ et $R_3$ représentent ensemble un groupe époxy et $R_4$ représente un groupe éthyle, appelé ici RGH—4478, ledit composé RGH—4478 ayant un point de fusion compris entre 180°C et 182°C, et un pouvoir rotatoire spécifique de $[\alpha]_D^{25} = +59{,}25°$ (c = 1, chloroforme),

produits qui sont éventuellement transformés ensuite en leurs sels d'addition acides.

6. Procédé tel que revendiqué dans l'une quelconque des revendications précédentes, dans lequel le mélange réactionnel, après réglage du pH, est hydrolysé et le mélange des dérivés N-desméthyle et N-desméthyl-N-formyle obtenus est soit séparé soit soumis à la formylation ce qui fait que le dérivé N-desméthyle est converti en le dérivé N-desméthyl-N-formyle.

7. Composé de formule Ia tel que défini dans la revendication 1, dans lequel $R_2$ représente un groupe éthyle, $R_3$ représente un atome d'hydrogène et $R_4$ représente un groupe hydroxyle, ledit composé ayant un point de fusion de 235°C à 238°C, et un pouvoir rotatoire spécifique de $[\alpha]_D^{25} = +30{,}5°$ (c = 1, chloroforme), et ses sels d'addition acides.

8. Compositions pharmaceutiques comprenant comme ingrédient actif, un composé de formule Ia tel que revendiqué dans la revendication 7 ou un de ses sels d'addition acides compatibles physiologiquement en association avec un véhicule ou un excipient pharmaceutique.

9. Composé tel que revendiqué dans la revendication 7 pour usage comme agent antitumoral.

**Revendications pour l'Etat contractant AT**

1. Procédé pour la préparation d'alcaloïdes de formule I

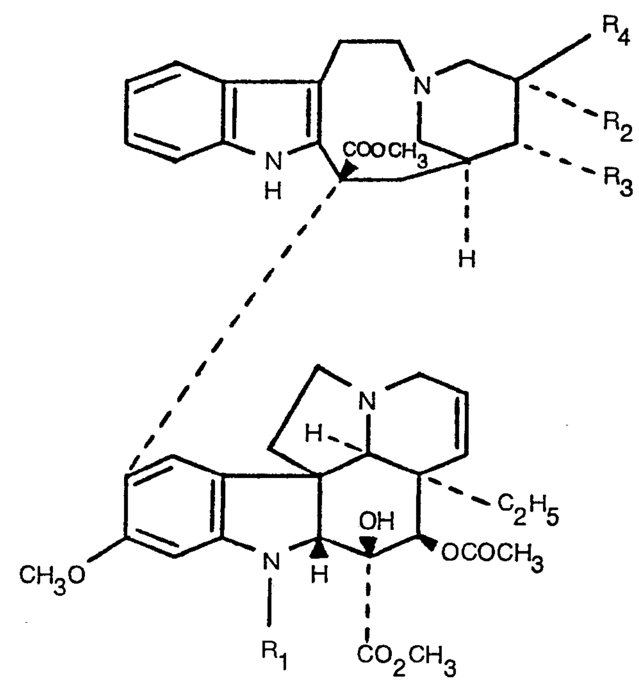

(I)

(dans laquelle $R_1$ représente un atome d'hydrogène ou un groupe formyle, et soit $R_2$ représente un groupe éthyle, $R_3$ représente un atome d'hydrogène et $R_4$ représente un groupe hydroxyle, soit $R_2$ et $R_3$ représentent ensemble un groupe époxy et $R_4$ représente un groupe éthyle) et leurs sels d'addition acides, qui consiste à faire réagir un alcaloïde de formule II

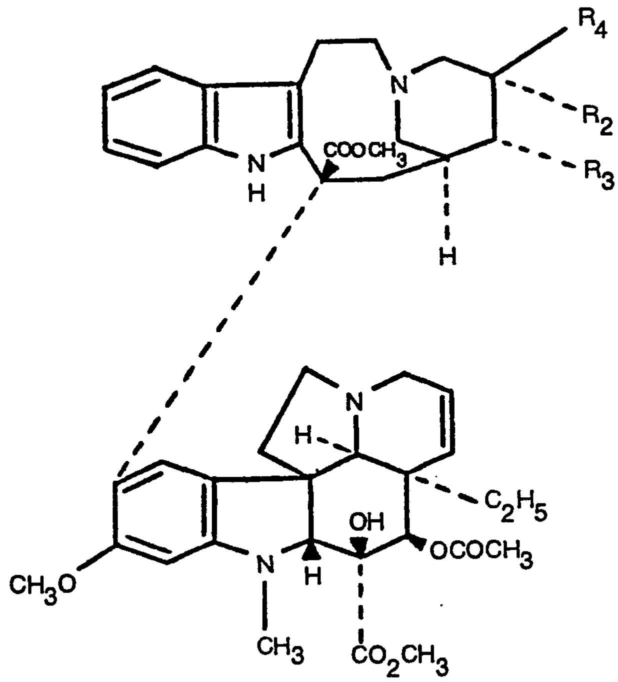

(II)

(dans laquelle $R_2$, $R_3$ et $R_4$ ont les définitions ci-dessus) ou un de ses sels d'addition acides avec l'acide chromique ou un bichromate de métal alcalin, à une température allant de −30°C à −90°C en présence d'un solvant organique non miscible avec l'eau dans lequel les matières de départ alcaloïdes et les produits alcaloïdes sont facilement solubles, d'anhydride acétique et éventuellement d'acide acétique glacial, le milieu réactionnel contenant également de l'éthanol à raison de 0,1 à 10% en volume, rapporté au volume du solvant organique non miscible avec l'eau; ensuite à régler le pH du mélange réactionnel entre 8 et 10; et à isoler les produits obtenus et/ou à soumettre lesdits produits à un traitement ultérieur; ce qui fait qu'en plus des composés de formule générale I, on obtient un composé de formule Ia

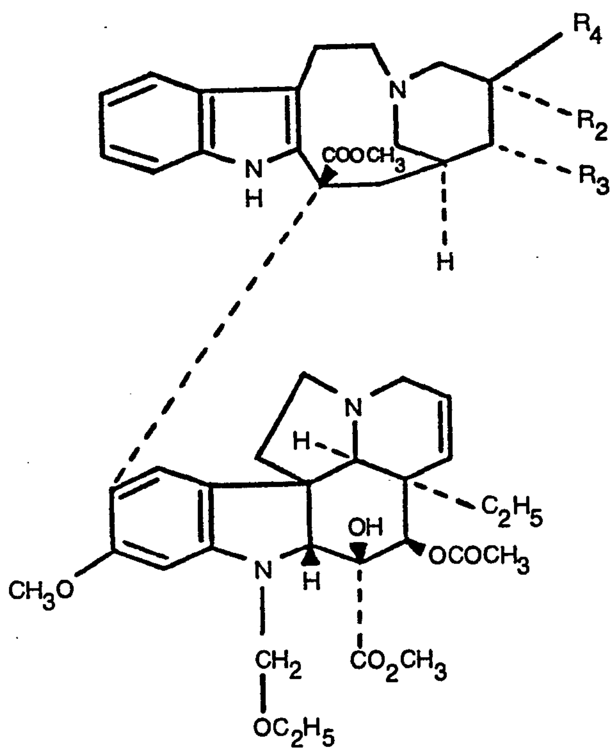

(Ia)

(dans laquelle $R_2$, $R_3$ et $R_4$ ont les définitions ci-dessus) ou bien un sel d'addition acide de ce composé.

2. Procédé tel que revendiqué dans la revendication 1, dans lequel le solvant organique non miscible avec l'eau est le chloroforme ou le chlorure de méthylène.

3. Procédé tel que revendiqué dans les revendications 1 ou 2, dans lequel l'oxydation est effectuée dans un milieu contenant de l'éthanolen une quantité allant de 0,1 à 3% en volume, rapporté au volume du solvant organique non miscible avec l'eau.

4. Procédé tel que revendiqué dans l'une quelconque des revendications précédentes, dans lequel la réaction d'oxydation est effectuée à une température allant de −45°C à −90°C.

5. Procédé tel que revendiqué dans l'une quelconque des revendications prédédentes, dans lequel on isole du mélange réactionnel après réglage du pH, soit

a) si la vinblastine ou un sel d'addition acide de celle-ci est utilisé(e) comme matière de départ: la N-desméthyl-vinblastine, la vincristine et un composé de formule Ia tel que défini dans la revendication 1, dans lequel $R_2$ représente un groupe éthyle, $R_3$ représente un atome d'hydrogène et $R_4$ représente un groupe hydroxyle, appelé ici RGH—4451, ledit composé RGH—4451 ayant un point de fusion compris entre 235°C et 238°C, et un pouvoir rotatoire spécifique de $[\alpha]_D^{25} = +30{,}5°$ (c = 1, chloroforme), soit

b) si la leurosine ou un sel d'addition acide de celle-ci est utilisé(e) comme matière de départ: la N-desméthyl-leurosine, la N-desméthyl-N-formyl-leurosine et un composé de formule Ia tel que défini dans la revendication 1, dans lequel $R_2$ et $R_3$ représentent ensemble un groupe époxy et $R_4$ représente un groupe éthyle, appelé ici RGH—4478, ledit composé RGH—4478 ayant un point de fusion compris entre 180°C et 182°C, et un pouvoir rotatoire spécifique de $[\alpha]_D^{25} = +59{,}25°$ (c = 1, chloroforme),

produits qu' sont éventuellement transformés ensuite en leurs sels d'addition acides.

6. Procédé tel que revendiqué dans l'une quelconque des revendications précédentes, dans lequel le mélange réactionnel, après réglage du pH, est hydrolysé et le mélange des dérivés N-desméthyle et N-desméthyl-N-formyle obtenus est soit séparé soit soumis à la formylation ce qui fait que le dérivé N-desméthyle est converti en le dérivé N-desméthyl-N-formyle.

7. Procédé tel que revendiqué dans la revendication 6, dans lequel l'hydrolyse est effectuée au moyen d'acide sulfurique aqueux, d'acide chlorhydrique aqueux ou d'acide acétique, à 0,5—5%.

8. Procédé tel que revendiqué dans chacune des revendications 6 et 7 dans lequel la formylation est effectuée au moyen d'un mélange d'acide formique et d'anhydride acétique.

**Patentansprüche für die Vertragsstaaten: BE CH DE FR GB IT LI LU NL SE**

1. Verfahren zur Herstellung von Alkaloiden der Formel I

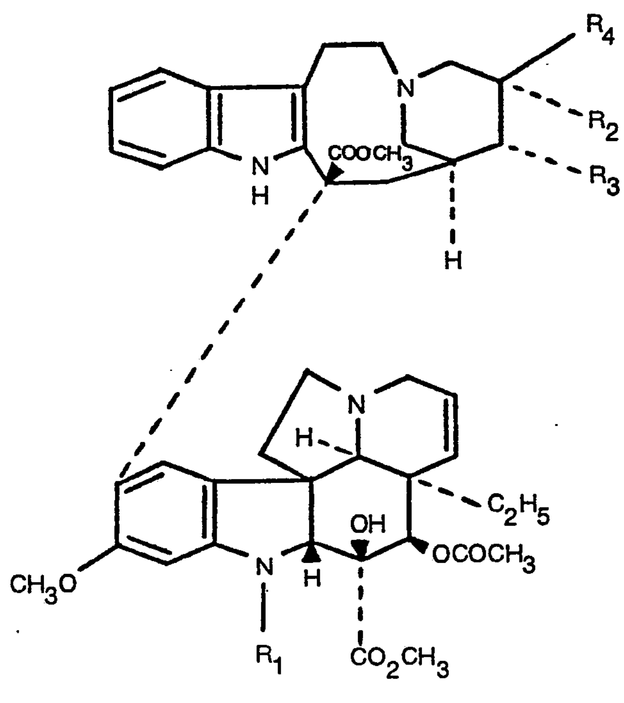

(I)

(worin $R_1$ ein Wasserstoffatom oder eine Formylgruppe und entweder $R_2$ eine Ethylgruppe, $R_3$ ein Wasserstoffatom und $R_4$ eine Hydroxylgruppe oder $R_2$ und $R_3$ zusammen eine Epoxygruppe und $R_4$ eine Ethylgruppe bedeuten) sowie von Säureadditionssalzen davon, dadurch gekennzeichnet, daß man ein Alkaloid der Formel II

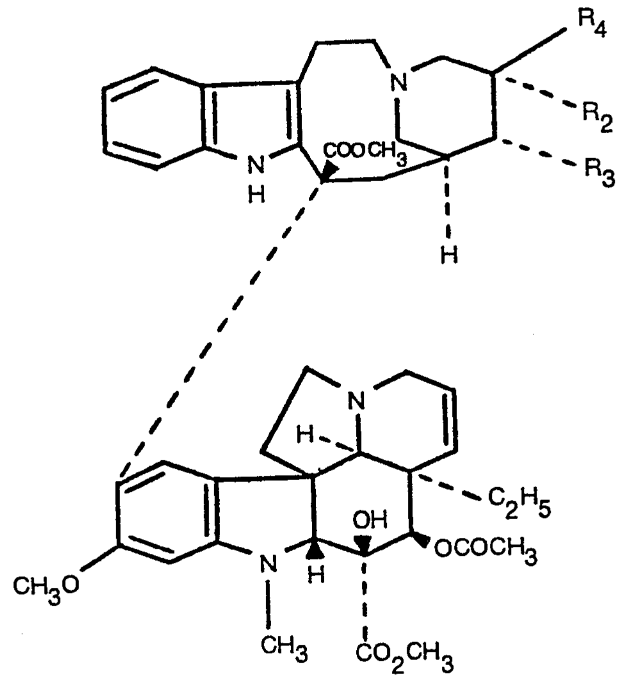

(II)

(worin $R_2$, $R_3$ und $R_4$ die oben angegebene Bedeutung besitzen) oder ein Säureadditionssalz davon mit Chromsäure oder einem Alkalidichromat bei einer Temperatur von —30 bis —90°C in Gegenwart eines mit Wasser unmischbaren organischen Lösungsmittels, in dem die als Ausgangsmaterial verwendeten Alkaloide sowie die Alkaloid-produkte leicht löslich sind, sowie von Acetanhydrid und gewünschtenfalls Eisessig, wobei das Reaktionsmedium ferner Ethanol in einer Menge von 0,1 bis 10%, bezogen auf das Volumen des mit Wasser unmischbaren organischen Lösungsmittels, enthält, umsetzt, danach den pH-Wert des Reaktionsgemisches auf 8 bis 10 einstellt und die erhaltenen Produkte isoliert und bzw. oder die Produkte einer weiteren Behandlung unterzieht, wobei zusätzlich zu den Verbindungen der allgemeinen Formel I eine Verbindung der Formel Ia

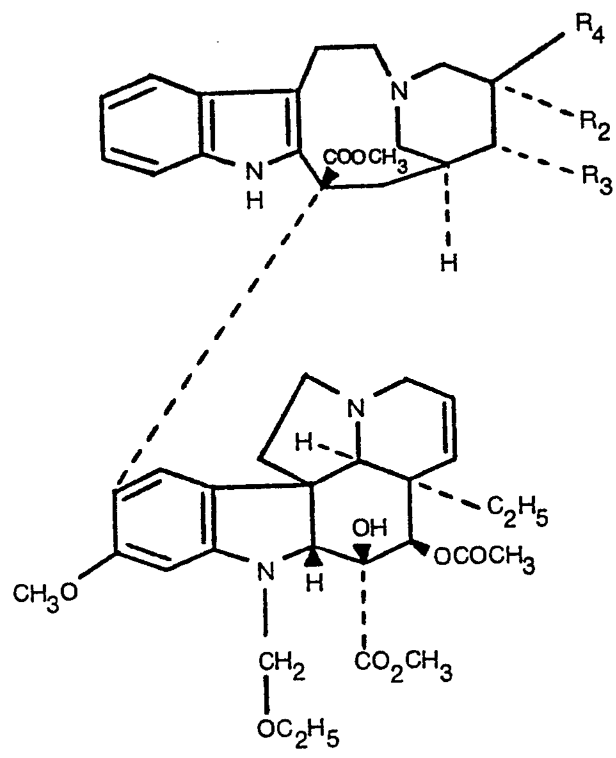

(Ia)

(worin $R_2$, $R_3$ und $R_4$ die oben angegebene Bedeutung besitzen) oder ein Säureadditionssalz davon erhalten wird.

2. Verfahren gemäß Anspruch 1, dadurch gekennzeichnet, daß das mit Wasser unmischbare Lösungsmittel Chloroform oder Methylenchlorid ist.

3. Verfahren gemäß Anspruch 1 oder 2, dadurch gekennzeichnet, daß die Oxidation in einem Medium durchgeführt wird, das Ethanol in einer menge von 0,1 bis 3,0%, bezogen auf das Volumen des mit Wasser unmischbaren organischen Lösungsmittels, enthält.

4. Verfahren gemäß einem der vorhergehenden Ansprüche, dadurch gekennzeichnet, daß die Oxidation bei einer Temperatur von —45 bis —90°C durchgeführt wird.

5. Verfahren gemäß einem der vorhergehenden Ansprüche, dadurch gekennzeichnet, daß aus dem Reaktionsgemisch nach Einstellung des pH-Wertes entweder,

a) wenn Vinblastin oder ein Säureadditionssalz davon als Ausgangsmaterial verwendet wird, N-Desmethyl-vinblastin, Vincristin und eine Verbindung der Formel Ia, wie in Anspruch 1 definiert, wobei $R_2$ eine Ethylgruppe, $R_3$ ein Wasserstoffatom und $R_4$ eine Hydroxylgruppe bedeuten, die als RGH—4451 bezeichnet wird und einen Schmelzpunkt von 235—238°C sowie eine spezifische Drehung von $[\alpha]_D^{25} = +30{,}5$ (c = 1, Chloroform) aufweist, oder,

b) wenn Leurosin oder ein Säureadditionssalz davon als Ausgangsmaterial verwendet wird, N-Desmethyl-leurosin, N-Desmethyl-N-formyl-leurosin und eine Verbindung der Formel Ia, wie in Anspruch 1 definiert, wobei $R_2$ und $R_3$ zusammen eine Epoxygruppe und $R_4$ eine Ethylgruppe bedeuten, die als RGH—4478 bezeichnet wird und einen Schmelzpunkt von 180—182°C sowie eine spezifische Drehung von $[\alpha]_D^{25} = +59{,}25°$ (c = 1, Chloroform) aufweist, isoliert werden, die gewünschtenfalls anschließend in ihre Säureadditionssalze umgewandelt werden.

6. Verfahren gemäß einem der vorhergehenden Ansprüche, dadurch gekennzeichnet, daß das Reaktionsgemisch nach Einstellen des pH-Wertes hydrolysiert und das erhaltene Gemisch aus N-Desmethyl- und N-Desmethyl-N-formyl-Derivaten entweder aufgetrennt oder der Formylierung unterworfen wird, wobei das N-Desmethyl-Derivat in das N-Desmethyl-N-formyl-Derivat umgewandelt wird.

7. Verbindung der Formel Ia, wie in Anspruch 1 definiert, wobei $R_2$ eine Ethylgruppe, $R_3$ ein Wasserstoffatom und $R_4$ eine Hydroxylgruppe bedeuten, mit einem Schmelzpunkt von 235 bis 238°C und einer spezifischen Drehung von $[\alpha]_D^{25} = +30{,}5°$ (c = 1, Chloroform) sowie Säureadditionssalze davon.

8. Pharmazeutische Präparate, enthaltend eine Verbindung der Formel Ia, wie in Anspruch 7 beansprucht, oder ein physiologisch verträgliches Säureadditionssalz davon als aktiven Bestandteil in Verbindung mit einem pharmazeutischen Träger oder Excipiens.

9. Verbindung gemäß Anspruch 7 zur Verwendung als antineoplastischer Wirkstoff.

**Patentansprüche für den Vertragsstaat AT**

1. Verfahren zur Herstellung von Alkaloiden der Formel I

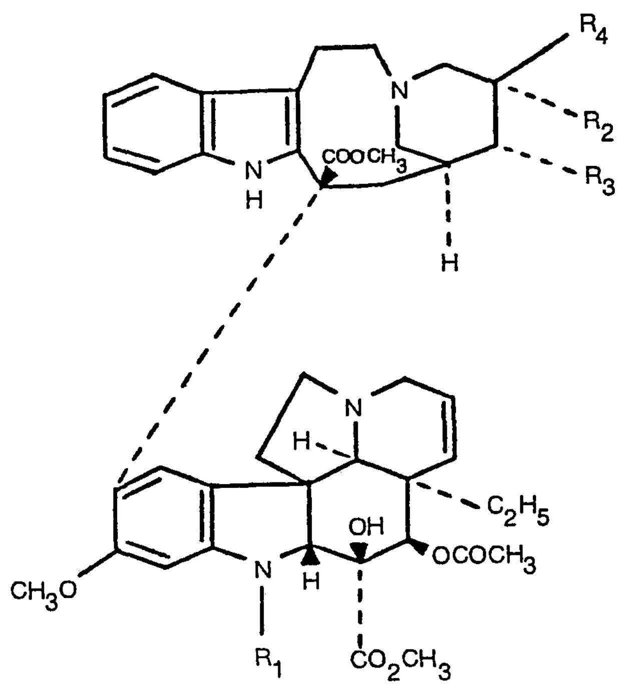

(I)

(worin $R_1$ ein Wasserstoffatom oder eine Formylgruppe und entweder $R_2$ eine Ethylgruppe, $R_3$ ein Wasserstoffatom und $R_4$ eine Hydroxylgruppe oder $R_2$ und $R_3$ zusammen eine Epoxygruppe und $R_4$ eine

Ethylgruppe bedeuten) sowie von Säureadditionssalzen davon, dadurch gekennzeichnet, daß man ein Alkaloid der Formel II

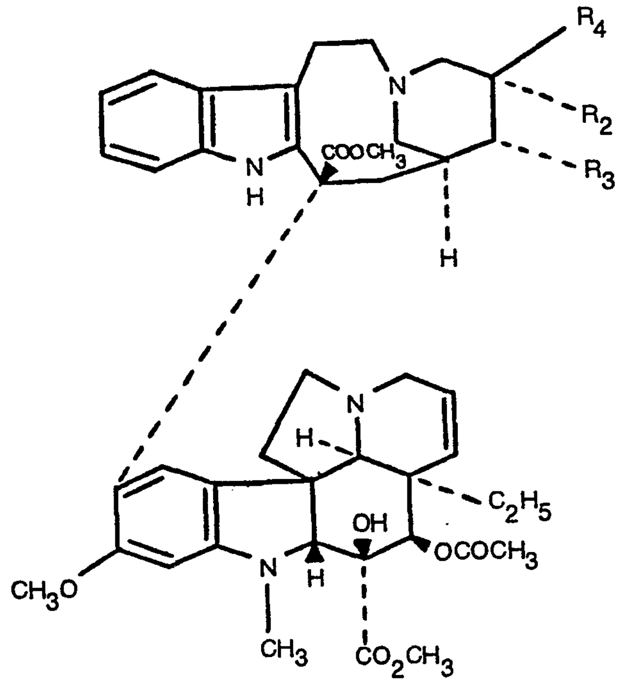

(II)

(worin $R_2$, $R_3$ und $R_4$ die oben angegebene Bedeutung besitzen) oder ein Säureadditionssalz davon mit Chromsäure oder einem Alkalidichromat bei einer Temperatur von −30 bis −90°C in Gegenwart eines mit Wasser unmischbaren organischen Lösungsmittels, in dem die als Ausgangsmaterial verwendeten Alkaloide sowie die Alkaloid-produkte leicht löslich sind, sowie von Acetanhydrid und gewünschtenfalls Eisessig, wobei das Reaktionsmedium ferner Ethanol in einer Menge von 0,1 bis 10%, bezogen auf das Volumen des mit Wasser unmischbaren organischen Lösungsmittels, enthält, umsetzt, danach den pH-Wert des Reaktionsgemisches auf 8 bis 10 einstellt und die erhaltenen Produkte isoliert und bzw. oder die Produkte einer weiteren behandlung unterzieht, wobei zusätzlich zu den Verbindungen der allgemeinen Formel I eine Verbindung der Formel Ia

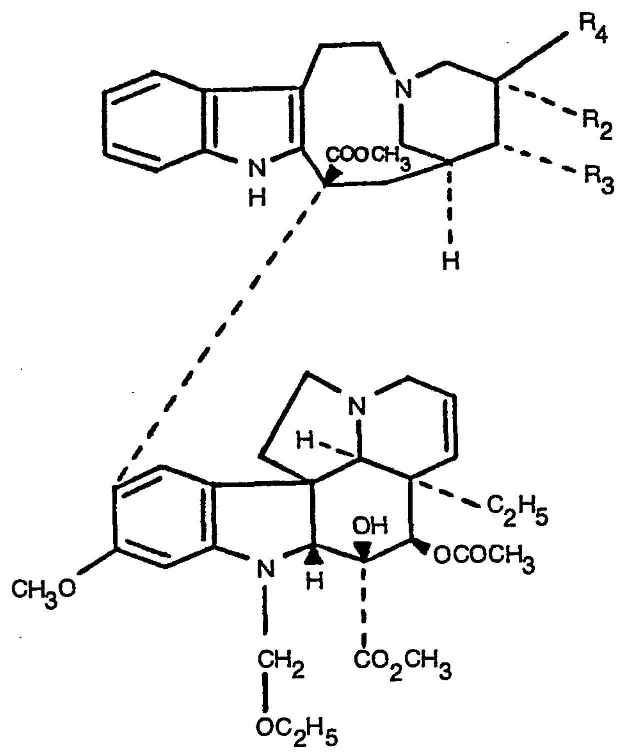

(Ia)

(worin $R_2$, $R_3$ und $R_4$ die oben angegebene Bedeutung besitzen) oder ein Säureadditionssalz davon erhalten wird.

2. Verfahren gemäß Anspruch 1, dadurch gekennzeichnet, daß das mit Wasser unmischbare Lösungsmittel Chloroform oder Methylenchlorid ist.

3. Verfahren gemäß Anspruch 1 oder 2, dadurch gekennzeichnet, daß die Oxidation in einem Medium durchgeführt wird, das Ethanol in einer menge von 0,1 bis 3,0%, bezogen auf das Volumen des mit Wasser unmischbaren organischen Lösungsmittels, enthält.

4. Verfahren gemäß einem der vorhergehenden Ansprüche, dadurch gekennzeichnet, daß die Oxidation bei einer Temperatur von —45 bis —90°C durchgeführt wird.

5. Verfahren gemäß einem der vorhergehenden Ansprüche, dadurch gekennzeichnet, daß aus dem Reaktionsgemisch nach Einstellung des pH-Wertes entweder,

a) wenn Vinblastin oder ein Säureadditionssalz davon als Ausgangsmaterial verwendet wird, N-Desmethyl-vinblastin, Vincristin und eine Verbindung der Formel Ia, wie in Anspruch 1 definiert, wobei $R_2$ eine Ethylgruppe, $R_3$ ein Wasserstoffatom und $R_4$ eine Hydroxylgruppe bedeuten, die als RGH—4451 bezeichnet wird und einen Schmelzpunkt von 235—238°C sowie eine spezifische Drehung von $[\alpha]_D^{25} = +30{,}5$ (c = 1, Chloroform) aufweist, oder,

b) wenn Leurosin oder ein Säureadditionssalz davon als Ausgangsmaterial verwendet wird, N-Desmethyl-leurosin, N-Desmethyl-N-formyl-leurosin und eine Verbindung der Formel Ia, wie in Anspruch 1 definiert, wobei $R_2$ und $R_3$ zusammen eine Epoxygruppe und $R_4$ eine Ethylgruppe bedeuten, die als RGH—4478 bezeichnet wird und einen Schmelzpunkt von 180—182°C sowie eine spezifische Drehung von $[\alpha]_D^{25} = +59{,}25°$ (c = 1, Chloroform) aufweist, isoliert werden, die gewünschtenfalls anschließend in ihre Säureadditionssalze umgewandelt werden.

6. Verfahren gemäß einem der vorhergehenden Ansprüche, dadurch gekennzeichnet, daß das Reaktionsgemisch nach Einstellen des pH-Wertes hydrolysiert und das erhaltene Gemisch aus N-Desmethyl- und N-Desmethyl-N-formyl-Derivaten entweder aufgetrennt oder der Formylierung unterworfen wird, wobei das N-Desmethyl-Derivat in das N-Desmethyl-N-formyl-Derivat umgewandelt wird.

7. Verfahren gemäß Anspruch 6, dadurch gekennzeichnet, daß die Hydrolyse mittels 0,5 bis 5%iger wässriger Schwefelsäure, Salzsäure oder Essigsäure bewirkt wird.

8. Verfahren gemäß einem der Ansprüche 6 und 7, dadurch gekennzeichnet, daß die Formylierung mit Hilfe eines Gemisches aus Ameisensäure und Acetanhydrid bewirkt wird.

FIG 1

RGH-4451-infrared spectrum

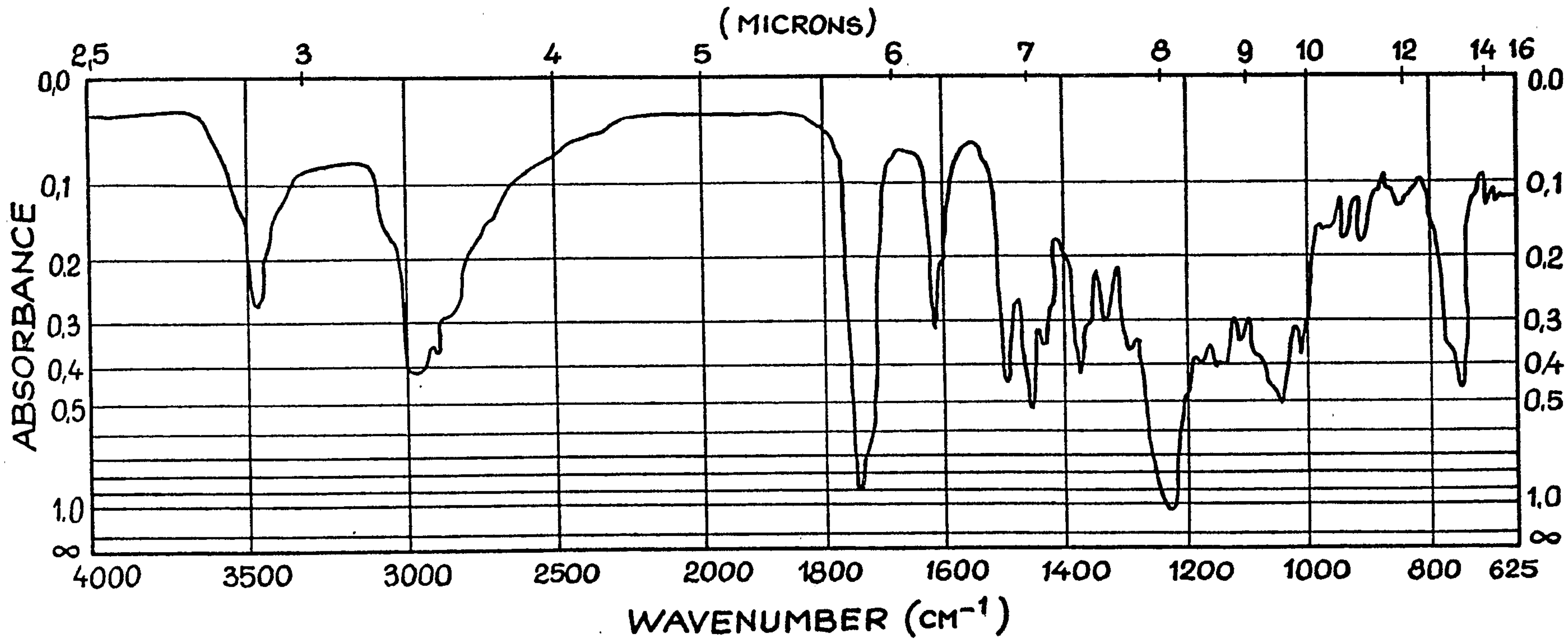

FIG 2

RGH-4451 $^1$H-NMR spectrum

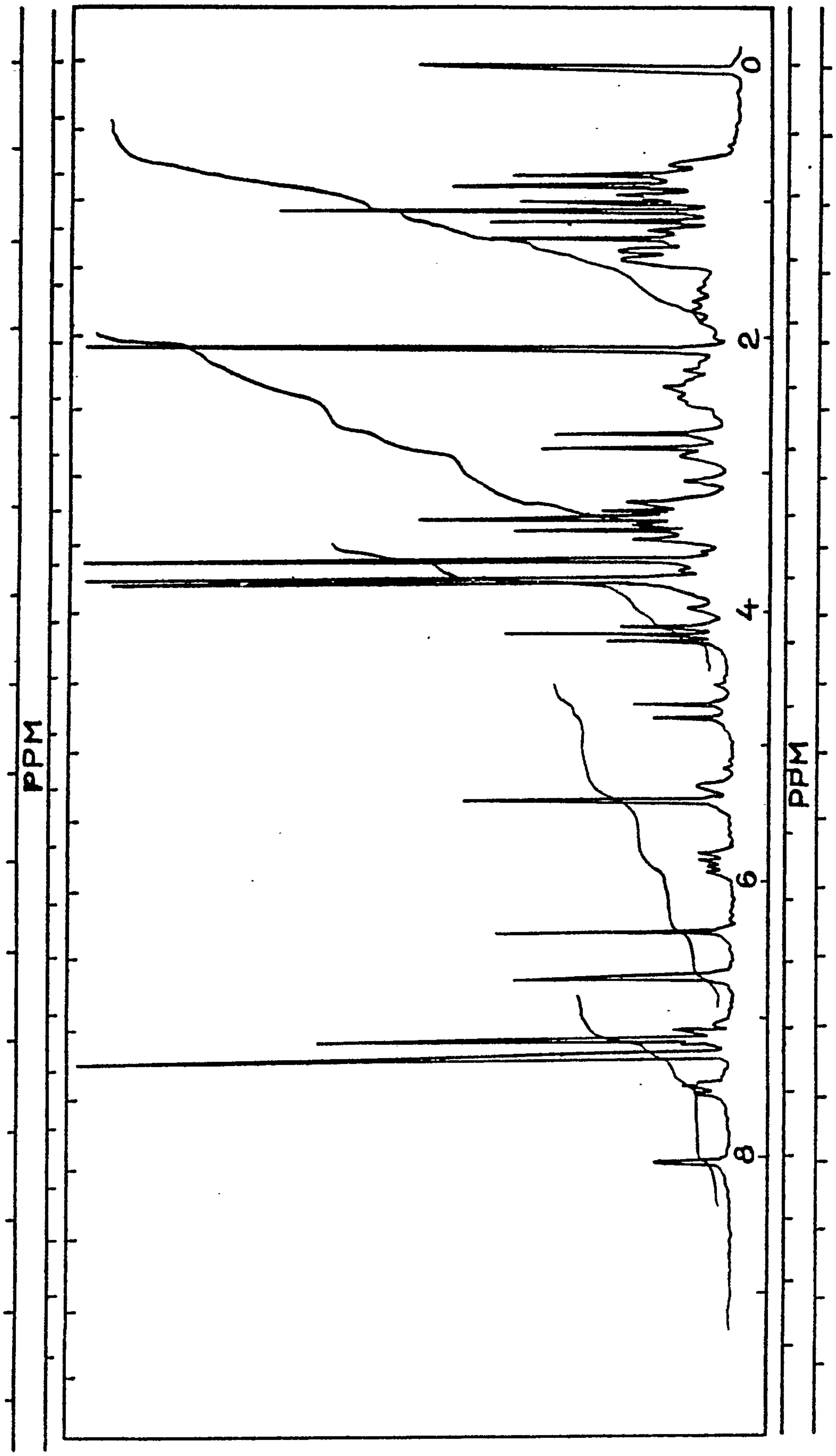

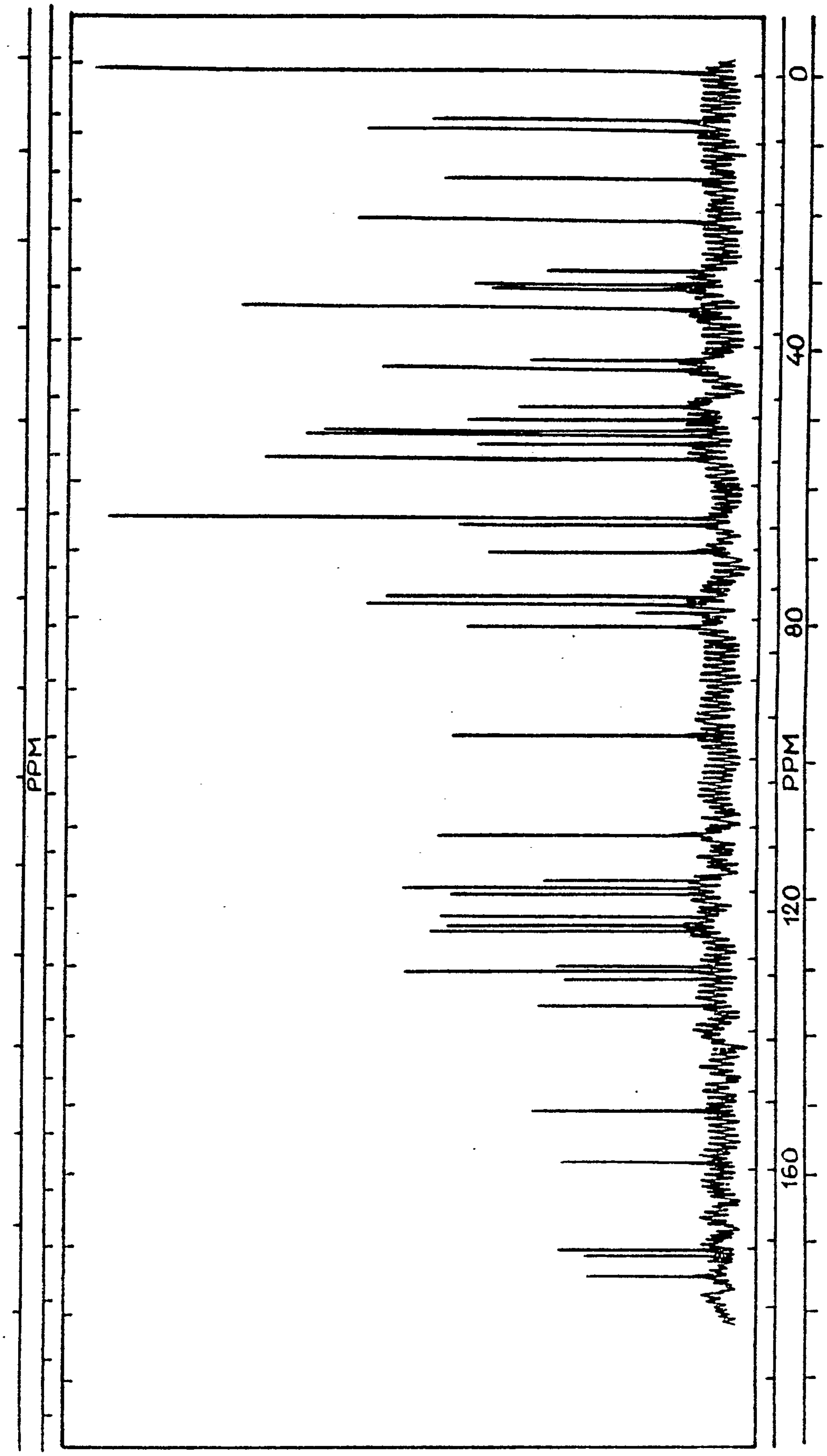


FIG 3

RGH-4451 $^{13}C$-NMR spectrum

FIG 4

RGH-4478 - infrared spectrum

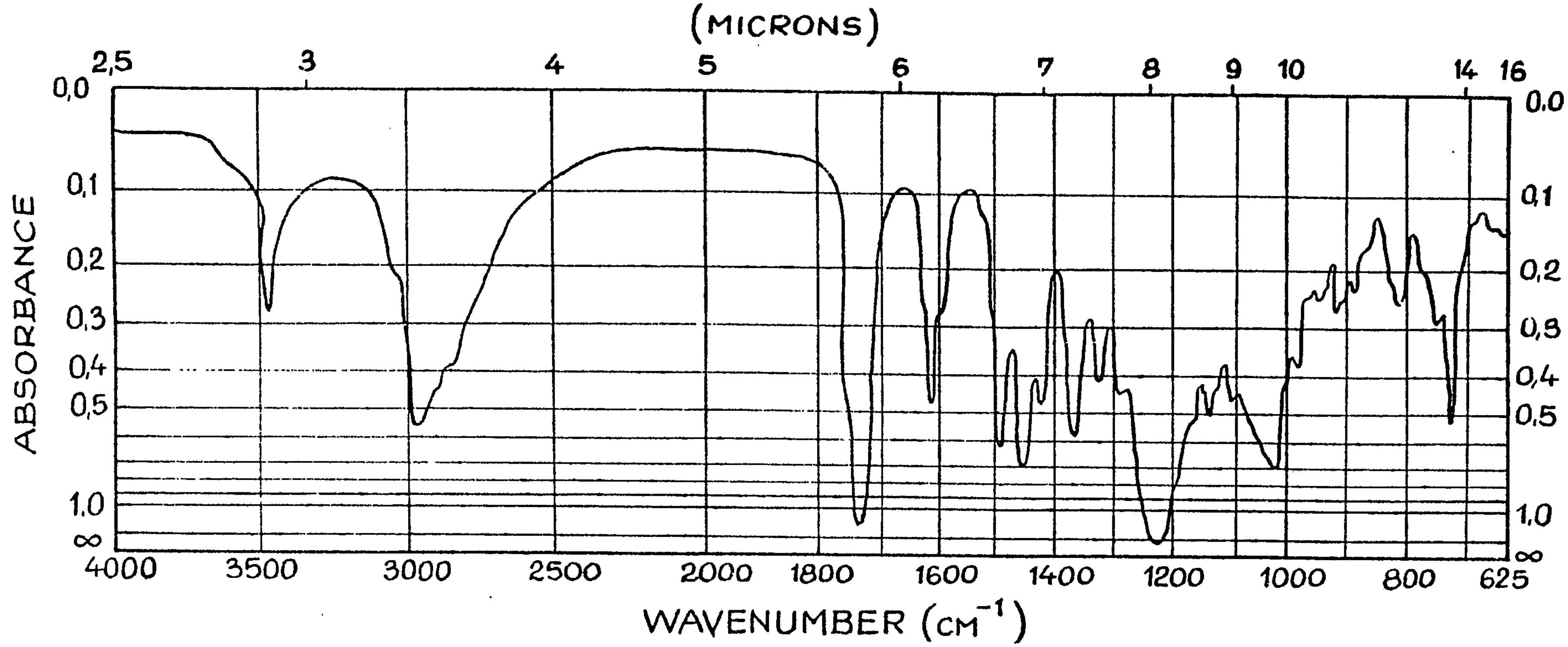

FIG 5

RGH-4478 $^1H$-NMR spectrum

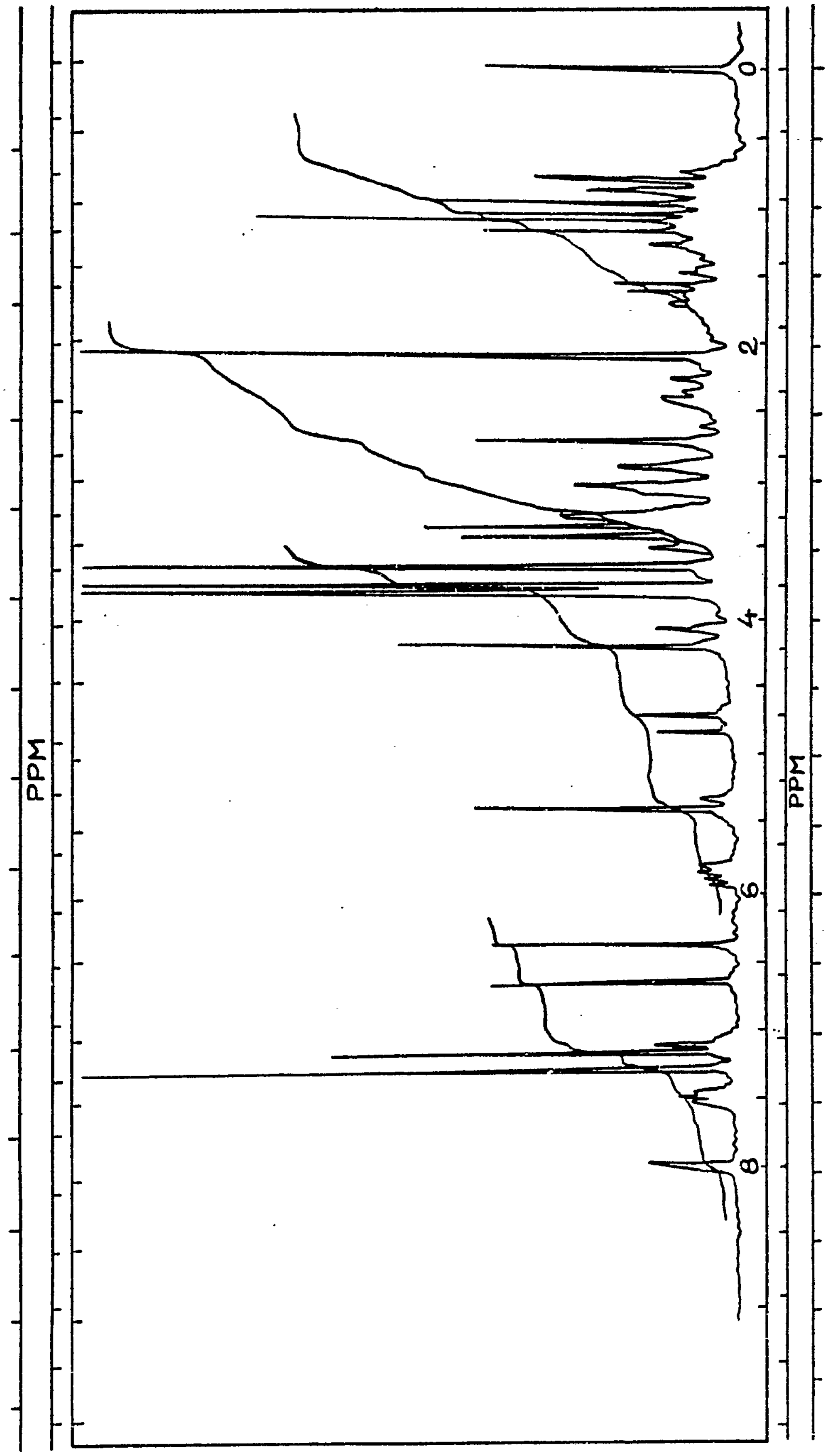

FIG 6

RGH-4478 $^{13}C$-NMR spectrum